(19) Europäisches Patentamt
European Patent Office
Office européen des brevets

(11) Veröffentlichungsnummer: **0 222 210 B1**

(12) **EUROPÄISCHE PATENTSCHRIFT**

(45) Veröffentlichungstag der Patentschrift: **28.03.90**

(51) Int. Cl.[4]: **C07F 9/651**, C07D 239/32, A01N 57/02

(21) Anmeldenummer: **86114482.2**

(22) Anmeldetag: **18.10.86**

(54) **Verfahren zur Herstellung von Phosphorsäurederivaten und Zwischenprodukten.**

(30) Priorität: **02.11.85 DE 3538912**

(43) Veröffentlichungstag der Anmeldung: **20.05.87 Patentblatt 87/21**

(45) Bekanntmachung des Hinweises auf die Patenterteilung: **28.03.90 Patentblatt 90/13**

(84) Benannte Vertragsstaaten: **BE CH DE FR GB IT LI NL**

(56) Entgegenhaltungen:
**EP-A- 0 167 888**
**FR-A- 2 365 577**

**D.J. BROWN et al.: "The pyrimidines", Band 16, Zusatz II, 1985, Seiten 252-253, John Wiley & Sons, New York, US**

(73) Patentinhaber: **BAYER AG, D-5090 Leverkusen 1 Bayerwerk(DE)**

(72) Erfinder: **Arold, Hermann, Dr., Falkenberg 151, D-5600 Wuppertal 1(DE)**
Erfinder: **Maurer, Fritz, Dr., Röberstrasse 8, D-5600 Wuppertal 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein neues Verfahren zur Herstellung von insektiziden Pyrimidinylphosphorsäurederivaten, Zwischenprodukte welche für die Durchführung des Verfahrens verwendet werden können, sowie Verfahren zur Herstellung solcher Zwischenprodukte.

Es ist bereits bekannt, daß man bestimmte pestizide Phosphorsäurepyrimidinester erhält, wenn man entsprechende Phosphorsäureesterchloride mit 5-Hydroxypyrimidinen umsetzt (vergl. DE-OS 2 643 262 und DE-OS 2 706 127). Diese Herstellungsmethode ist jedoch wegen des Fehlens geeigneter Ausgangsverbindungen bzw. wegen unbefriedigender Herstellungsmethoden hierfür nur begrenzt anwendbar. Es besteht daher Bedarf an neuen Zwischenprodukten und ein entsprechendes Herstellungsverfahren für Phosphorsäurepyrimidinester. Ein mehrstufiges Verfahren zur Herstellung von Phosphorsäurepyrimidinestern wird in der älteren, nicht vorveröffentlichten, EP-A 167 888 beschrieben. Ein Verfahren zur Acylierung eines 5-Hydroxypyrimidins unter Erhaltung von 2- und 4-Hydroxy-Gruppen ist aus The Pyrimidines, Band 16, Zusatz II, 1985, Seiten 252 bis 253 bekannt.

Es wurde nun gefunden, daß man die Verbindungen der allgemeinen Formel (I)

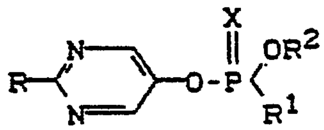

(I)

in welcher
R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$–$C_4$-Alkoxy oder $C_1$–$C_4$-Alkylsulfonyl substituiertes Alkyl mit 1 bis 12 Kohlenstoffatomen, für gegebenenfalls durch $C_1$–$C_4$-Alkyl substituiertes Cycloalkyl mit 3 bis 8 Kohlenstoffatomen, und für gegebenenfalls durch $C_1$–$C_4$-Alkyl, $C_1$–$C_4$-Alkoxy oder $C_1$–$C_4$-Alkylsulfonyl substituiertes Aryl mit 6 bis 10 Kohlenstoffatomen,
$R^1$ für gegebenenfalls durch $C_1$–$C_4$-Alkyl (gilt nicht, wenn $R^1$ für Alkyl steht), $C_1$–$C_4$-Alkoxy, $C_1$–$C_4$-Alkylthio, Halogen, Cyano und/oder Nitro substituierte Reste aus der Reihe $C_1$–$C_6$-Alkyl, $C_1$–$C_6$-Alkoxy, $C_1$–$C_6$-Alkylthio, Mono- oder Di-($C_1$–$C_5$)-alkylamino und Phenyl steht,
$R^2$ für gegebenenfalls durch $C_1$–$C_4$-Alkoxy, $C_1$–$C_4$-Alkylthio, Halogen, Cyano und/oder Nitro substituiertes $C_1$–$C_6$-Alkyl steht und
X für Sauerstoff oder Schwefel steht,
erhält, wenn man

a) Verbindungen der allgemeinen Formel (II)

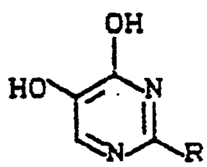

(II)

in welcher
R die oben angegebenen Bedeutungen hat,
mit Acylierungsmitteln der Formel (III)

$R^3$–CO–Y (III)

in welcher
$R^3$ für $C_1$–$C_4$-Alkyl, $C_1$–$C_4$-Alkoxy oder Phenyl steht und
Y für Halogen oder eine Gruppierung –OCOR$^3$ steht,
gegebenenfalls in Gegenwart von Säureakzeptoren und gegebenenfalls in Gegenwart von Verdünnungsmitteln bei Temperaturen zwischen 0°C und 160°C zu den Verbindungen der allgemeinen Formel (IV)

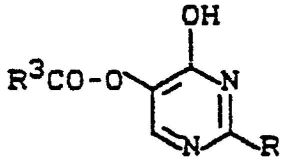

(IV)

in welcher
R und $R^3$ die oben angegebenen Bedeutungen haben,
umsetzt und anschließend

b) die Verbindungen der allgemeinen Formel (IV), gegebenenfalls nach ihrer Isolierung mit Halogenierungsmitteln gegebenenfalls in Gegenwart von N,N-disubstituierten Amiden als Katalysatoren und gegebenenfalls in Gegenwart von Verdünnungsmitteln bei Temperaturen zwischen 10°C und 120°C zu den Verbindungen der allgemeinen Formel (V)

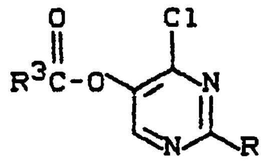

(V)

in welcher
R und $R^3$ die oben angegebenen Bedeutungen haben,
umsetzt und anschließend

c) die Verbindungen der allgemeinen Formel (V), gegebenenfalls nach ihrer Isolierung, in Gegenwart von anorganischen Basen bei Temperaturen zwischen 0°C und 160°C zu den Salzen der Verbindungen der allgemeinen Formel (VI)

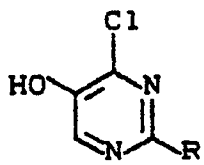

(VI)

in welcher
R die oben angegebene Bedeutung hat,
mit den verwendeten Basen umsetzt und gegebenenfalls nach Freisetzung der Verbindung der allgemeinen Formel (VI) durch Ansäuren anschließend

d) die Verbindungen der allgemeinen Formel (VI) oder ihre Salze, gegebenenfalls nach ihrer Isolierung, mit Wasserstoff in Gegenwart von Hydrierungskatalysatoren, gegebenenfalls in Gegenwart von Säureakzeptoren und in Gegenwart von Verdünnungsmitteln, bei Temperaturen zwischen 20 °C und 150 °C zu den Verbindungen der allgemeinen Formel (VII)

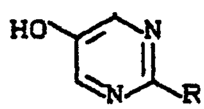

(VII)

in welcher
R die oben angegebene Bedeutung hat,
oder ihre Salze (mit den bei Stufe c) eingesetzten Basen) umsetzt und gegebenenfalls nach Freisetzung der Verbindungen der allgemeinen Formel (VII) durch Ansäuren anschließend

e) die Verbindungen der allgemeinen Formel (VII) oder ihre Salze gegebenenfalls nach ihrer Isolierung, mit Verbindungen der allgemeinen Formel (VIII)

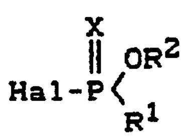

(VIII)

in welcher
Hal für Halogen steht und
X, $R^1$ und $R^2$ die oben angegebenen Bedeutungen haben,
gegebenenfalls in Gegenwart eines Säurebindemittels, gegebenenfalls in Gegenwart eines bicyclischen organischen Amins und gegebenenfalls in Gegenwart eines Lösungsmittels, umsetzt und die Verbindungen der allgemeinen Formel (I) isoliert.

Gemäß diesem Verfahren ist es möglich, die Verbindungen der Formel (I) auf einfache Weise in guter Reinheit und Ausbeute herzustellen. Das Verfahren ist im Hinblick auf die Art der gewünschten Substituenten sehr breit anwendbar. Weiterhin sind die als Zwischenprodukte einzusetzenden Verbindungen stabil und können gut gelagert und gehandhabt werden.

Bevorzugte Substituenten bzw. Bereiche der in den oben und nachfolgend erwähnten Formeln aufgeführten Reste werden im folgenden erläutert:

Alkoxy R steht für geradkettiges oder verzweigtes Alkoxy mit 1 bis 12, insbesondere 1 bis 6 und besonders bevorzugt 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Mono- oder Di-Alkylamino R steht für eine Aminogruppe mit 1 oder 2 Alkylgruppen, vorzugsweise 2 Alkylgruppen, welche jeweils geradkettig oder verzweigt sein können und 1 bis 5, insbesondere 1 bis 3 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Als gegebenenfalls substituiertes Alkyl R steht geradkettiges oder verzweigtes Alkyl mit 1 bis 12, insbesondere 1 bis 6 und besonders bevorzugt 1 bis 4 Kohlenstoffatomen. Beispielhaft seien gegebenenfalls substituiertes Methyl, Ethyl, n-Propyl, i-Propyl, n-Butyl, sec.-Butyl, i-Butyl, tert.-Butyl, n-Pentyl, i-Pentyl und tert.-Pentyl genannt.

Als gegebenenfalls substituiertes Cycloalkyl R steht Cycloalkyl mit 3 bis 8, insbesondere 3, 5 oder 6 Kohlenstoffatomen. Beispielhaft seien gegebenenfalls substituiertes Cyclopropyl, Cyclobutyl, Cyclopentyl, Cyclohexl und Cycloheptyl genannt.

Als gegebenenfalls substituiertes Aryl R steht Aryl mit 6 bis 10 Kohlenstoffatomen im Arylteil. Beispielhaft seien gegebenenfalls substituiertes Phenyl oder Naphthyl, insbesondere Phenyl genannt.

Die in der Definition von R genannten substituierten Reste können einen oder mehrere, vorzugsweise 1 bis 3, insbesondere 1 oder 2 gleiche oder verschiedene Substituenten tragen. Als Substituenten für Alkyl, Cycloalkyl und Aryl seien aufgeführt:

Alkoxy und Alkylsulfonyl mit 1 bis 4 Kohlenstoffatomen wie Methoxy, Ethoxy, n-Propoxy, i-Propoxy, n-Butoxy, i-Butoxy, sec.-Butoxy, tert.-Butoxy, Methylsulfonyl, Ethylsulfonyl, n-Propylsulfonyl, i-Propylsulfonyl, n-Butylsulfonyl, i-Butylsulfonyl und tert.-Butylsulfonyl.

Als Arylsubstituenten und Cycloalkylsubstituenten kann außerdem noch $C_1$–$C_4$-Alkyl stehen, wie Methyl, Ethyl, n-Propyl, i-Propyl, n-Butyl, i-Butyl, sec.-Butyl und tert.-Butyl.

Besonders bevorzugt steht

R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen, und für gegebenenfalls durch Methyl, Ethyl, Methoxy, Ethoxy, Methylsulfonyl oder Ethylsulfonyl substituiertes Phenyl.

Ganz besonders bevorzugt steht

R für Methyl, Isopropyl und tert.-Butyl, insbesondere für Isopropyl und tert.-Butyl.

Die gegebenenfalls substituierten Alkylgruppen $R^1$ und $R^2$ enthalten 1 bis 6, insbesondere 1 bis 4 und besonders bevorzugt 1 oder 2 Kohlenstoffatome. Beispielhaft seien Methyl, Ethyl, n- und i-Propyl, n-, i-, s- und t-Butyl genannt.

Die Alkylgruppen der gegebenenfalls substituierten Alkyl- und Dialkylaminogruppen $R^1$ haben die für die Alkylgruppen $R^1$ und $R^2$ oben angegebene Bedeutung. Beispielhaft seien Methyl-, Ethyl-, n- und i-Propylamino sowie Dimethyl-, Diethyl- und Methyl-ethyl-amino aufgeführt.

Die Alkoxy- und Alkylthioreste $R^1$ enthalten 1 bis 6, insbesondere 1 bis 4 und besonders bevorzugt 1 oder 2 Kohlenstoffatome. Beispielhaft seien Methoxy, Ethoxy, n- und i-Propoxy sowie Methylthio, Ethylthio und n- und i-Propylthio genannt.

Die gegebenenfalls substituierten Reste $R^1$ und $R^2$ können einen oder mehrere, vorzugsweise 1 bis 3, insbesondere 1 oder 2 gleiche oder verschiedene Substituenten tragen. Als Substituenten seien aufgeführt: Alkyl (gilt nicht für die Fälle in welchen $R^1$ bzw. $R^2$ für Alkyl steht) mit 1 bis 4, insbesondere 1 oder 2 Kohlenstoffatomen wie Methyl, Ethyl, n- und i-Propyl, und n-, i-, s- und t-Butyl; Alkoxy mit 1 bis 4, insbesondere 1 oder 2 Kohlenstoffatomen wie Methoxy, Ethoxy, n- und i-Propyloxy und n-, i-, s- und t-Butyloxy; Alkylthio mit 1 bis 4, insbesondere 1 oder 2 Kohlenstoffatomen wie Methylthio, Ethylthio, n- und i-Propylthio und n-, i-, s- und t-Butylthio; Halogen, vorzugsweise Fluor, Chlor, Brom und Iod, insbesondere Chlor und Brom; Cyano und Nitro.

$R^1$ steht für Ethoxy oder sec.-Butoxy. $R^2$ steht vorzugsweise für Ethyl. X steht vorzugsweise für Schwefel.

Alkyl $R^3$ steht für $C_1$–$C_4$-Alkyl, insbesondere für $C_1$–$C_2$-Alkyl, wobei beispielhaft Methyl, Ethyl, n- und i-Propyl sowie n-, i-, s- und t-Butyl (besonders bevorzugt Methyl) genannt seien.

Alkoxy $R^3$ steht für $C_1$–$C_4$-Alkoxy, insbesondere für $C_1$–$C_2$-Alkoxy, wobei beispielhaft Methoxy, Ethoxy, n- und i-Propoxy sowie n-, i-, s- und t-Butoxy genannt seien.

Besonders bevorzugt steht $R^3$ für Methyl.

Halogen Y und Hal bedeutet (wo nicht anders erläutert) Fluor, Chlor, Brom und Iod, vorzugsweise Fluor, Chlor und Brom, insbesondere Chlor.

Im Verfahrensschritt (a) werden die Verbindungen der allgemeinen Formel (II) gegebenenfalls ohne ihre Isolierung in die neuen Verbindungen der allgemeinen Formel (IV) umgewandelt. Die Verbindungen der allgemeinen Formel (IV) sind Teil der vorliegenden Erfindung.

Es wurde gefunden, daß man die neuen 4-Hydroxy-pyrimidin-Derivate der allgemeinen Formel (IV)

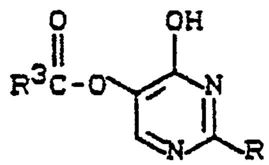

(IV)

in welcher

R und $R^3$ die oben angegebenen Bedeutungen haben, erhält, wenn man 4,5-Dihydroxy-pyrimidine der allgemeinen Formel (II)

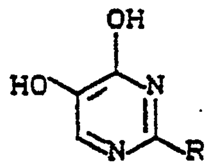

(II)

in welcher

R die oben angegebenen Bedeutungen hat,

mit Acylierungsmitteln der Formel (III)

$R^3$–CO–Y (III)

in welcher

$R^3$ und Y die oben angegebenen Bedeutungen haben,

gegebenenfalls in Gegenwart von Säureakzeptoren und gegebenenfalls in Gegenwart von Verdünnungsmitteln bei Temperaturen zwischen 0°C und 160°C umsetzt.

Verwendet man beim Verfahrensschritt (a) 4,5-Dihydroxy-2-methyl-pyrimidin als Ausgangsstoff und Essigsäureanhydrid als Acylierungsmittel, so kann die Reaktion durch das folgende Formelschema skizziert werden:

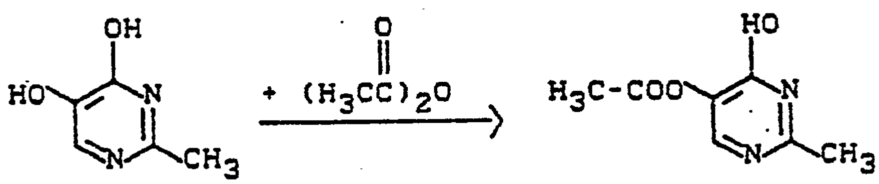

Als Beispiele für die Ausgangsverbindungen der allgemeinen Formel (II) seien die folgenden Verbindungen aufgeführt:

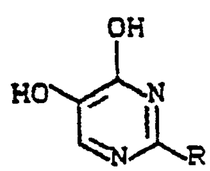

(II)

Tabelle 1

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 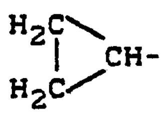 | 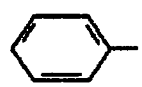 |
| 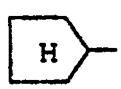 | 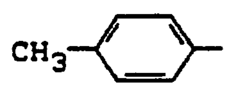 |
| 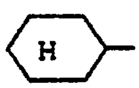 | |

Die erfindungsgemäß einzusetzenden Verbindungen der Formel (II) sind bekannt und/oder lassen sich in einfacher Weise nach bekannten Methoden herstellen, aus 5-Alkoxy-4-hydroxy-pyrimidinen der Formel (IX)

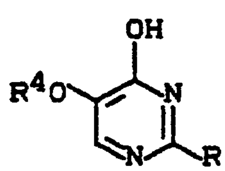

(IX)

in welcher
R die oben angegebenen Bedeutungen hat und
$R^4$ für $C_1$-$C_4$-Alkyl steht,
und starken Säuren wie z. B. Bromwasserstoffsäure oder konzentrierter Salzsäure bei Temperaturen zwischen 20 °C und 140 °C (vergl. J. Chem. Soc. 1963, 5590 und die Herstellungsbeispiele).

Die Verbindungen der Formel (IX) sind bekannt und/oder lassen sich nach bekannten Methoden herstellen (vergl. DE-OS 2 639 256).

Als Beispiele für die Verbindungen der Formel (III) seien genannt:
Essigsäureanhydrid, Acetylfluorid, Acetylchlorid, Acetylbromid, Benzoylchlorid, Benzoesäureanhydrid, Chlorameisensäuremethylester, Chlorameisensäureethylester, Chlorameisensäure-n-butylester, Pyrokohlensäuredimethylester und Pyrokohlensäurediethylester. Als besonders bevorzugt seien Essigsäureanhydrid oder Acetylchlorid genannt.

Die Verbindungen der Formel (III) sind allgemein bekannte Verbindungen der organischen Chemie.

Der Verfahrensschritt (a) wird bevorzugt in Gegenwart von Verdünnungsmitteln durchgeführt. Als Verdünnungsmittel kommen vorzugsweise in Frage:
aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methylisobutylketon, Ester wie Essigsäuremethylester und -ethylester, Nitrile wie z.B. Acetonitril und Propionitril, Amide wie z.B. Dimethylacetamid und N-Methyl-pyrrolidon sowie Tetramethylensulfon.

Als Basen für den Verfahrensschritt (a) können praktisch alle üblicherweise verwendbaren Säurebindemittel eingesetzt werden. Hierzu gehören insbesondere:
Alkali- und Erdalkalihydroxide, bzw. -oxide wie Natrium- und Kaliumhydroxide und insbesondere Lithiumhydroxid sowie Calciumoxid oder Calcium-hydroxid, Alkali- und Erdalkalicarbonate wie Natrium-, Kalium und Calciumcarbonate, Alkalialkoholate wie Natrium- oder Kalium-tert.-butylat, ferner aliphatische, aromatische oder heterocylische Amine wie Triethylamin, Dimethylanilin, Dimethylbenzylamin, Pyridin, Diazabicyclooctan und Diazabicycloundecen. Beim Einsatz von Säureanhydriden als Acylierungsmittel wird bevorzugt ohne Säureakzeptor gearbeitet.

Die Reaktionstemperatur kann innerhalb eines größeren Bereichs variiert werden. Im allgemeinen arbeitet man zwischen 0°C und 160°C, vorzugsweise bei 20°C bis 140°C. Das erfindungsgemäße Verfahren wird im allgemeinen bei Normaldruck durchgeführt.

Zur Durchführung des Verfahrensschritts (a) werden die Ausgangsstoffe gewöhnlich in etwa äquimolaren Mengen eingesetzt. Ein Überschuß der einen oder anderen Reaktionskomponente bringt keine wesentlichen Vorteile. Die Aufarbeitung sowie die gegebenenfalls gewünschte Isolierung geschieht nach üblichen Methoden.

Als Beispiele für die Verbindungen der Formel (IV) seien die folgenden Verbindungen aufgeführt:

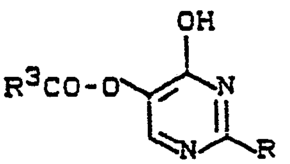

(IV)

$R^3$= $CH_3$-, $C_2H_5$-, n-$C_3H_7$-, 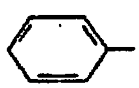 , $CH_3O$-, $C_2H_5O$-, n-$C_4H_9O$-

Tabelle 2

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 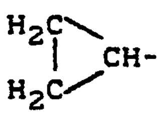 | 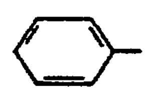 |
| 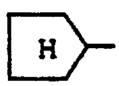 | 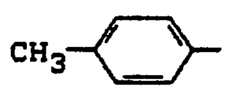 |
| 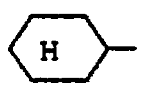 | |

Im Verfahrensschritt (b) werden die Verbindungen der allgemeinen Formel (IV) gegebenenfalls ohne ihre Isolierung in die neuen Verbindungen der allgemeinen Formel (V) umgewandelt. Die Verbindungen der allgemeinen Formel (V) sowie das Verfahren zu ihrer Herstellung gemäß Verfahrensschritt (b) sind Teil der vorliegenden Erfindung.

Es wurde somit gefunden, daß man die neuen 4-Chlor-pyrimidin-Derivate der allgemeinen Formel (V)

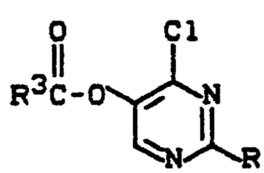 (V)

in welcher

R und $R^3$ die oben angegebenen Bedeutungen haben,

erhält, wenn man 4-Hydroxy-pyrimidin-Derivate der allgemeinen Formel (IV)

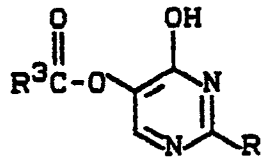

(IV)

in welcher
R und $R^3$ die oben angegebenen Bedeutungen haben,
mit Halogenierungsmitteln gegebenenfalls in Gegenwart von N,N'-disubstituierten Amiden als Katalysatoren und gegebenenfalls in Gegenwart von Verdünnungsmitteln bei Temperaturen zwischen 10 °C und 120 °C umsetzt.

Überraschenderweise kann man die neuen 4-Chlor-pyrimidin-Derivate der allgemeinen Formel (V) erfindungsgemäß auf einfache Weise erhalten, obwohl man hätte erwarten müssen, daß unter den Reaktionsbedingungen auch Seitenkettenreaktionen und/oder Abspaltungsreaktionen unter Bildung von z. B. Säurechloriden stattfinden bzw. bei dem Einsatz von substituierten Amiden z. B. Formylpyrimidine ("Vilsmeyer-Reaktion") entstehen.

Verwendet man beispielsweise bei dem erfindungsgemäßen Verfahren bzw. dem Verfahrensschritt (b) 4-Hydroxy-2-methyl-5-methylcarbonyloxy-pyrimidin als Ausgangsstoff und Phosphoroxychlorid als Halogenierungsmittel, so kann die Reaktion durch das folgende Formelschema skizziert werden:

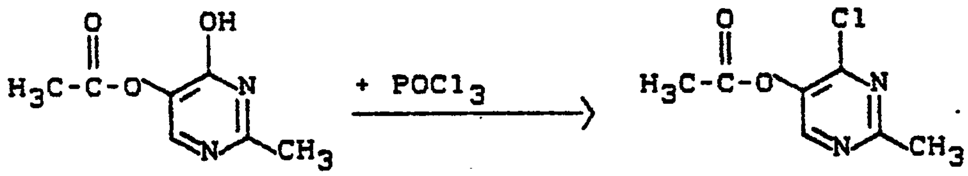


Das erfindungsgemäße Verfahren zur Herstellung der Verbindungen der allgemeinen Formel (V) bzw. der Verfahrensschritt (b) wird bevorzugt in Gegenwart von Verdünnungsmitteln durchgeführt. Als Verdünnungsmittel kommen inerte organische Lösungsmittel, wie gegebenenfalls halogenierte aliphatische oder aromatische Kohlenwasserstoffe sowie polare Lösungsmittel, z. B. Amide, in Frage. Hierzu gehören: Benzol, Toluol, Xylol, Chlorbenzol, o-Dichlorbenzol, Methylenchlorid, Ethylenchlorid, Chloroform, Tetrachlorkohlenstoff, Amide wie z.B. Dimethylformamid, N-Methylformamid und N-Methylpyrrolidon.

Als Halogenierungsmittel für das erfindungsgemäße Verfahren werden die üblichen Halogenierungsmittel verwendet, vorzugsweise: Phosphoroxychlorid, Phosphortrichlorid, Phosphorpentachlorid, Oxalylchlorid, Phosgen oder Thionylchlorid.

Das Verfahren bzw. der Verfahrensschritt (b) kann in Gegenwart von N,N-disubstituierten Amiden als Katalysatoren durchgeführt werden. Als N,N-disubstituierte Amide kommen dabei vorzugsweise in Frage: Dimethylformamid, N-Methylformanilid, N-Methylpyrrolidon oder N-Methylpiperidon.

Das erfindungsgemäße Verfahren bzw. der Verfahrensschritt (b) wird im allgemeinen bei Temperaturen zwischen 10 °C und 120 °C durchgeführt. Bevorzugt wird der Bereich zwischen 20 °C und 100 °C. Die Umsetzungen werden im allgemeinen bei Normaldruck durchgeführt.

Zur Durchführung des erfindungsgemäßen Verfahren bzw. des Verfahrensschritts (b) werden auf 1 Mol der Verbindung der Formel (IV), 1 bis 3 Mol, vorzugsweise1,1 bis 2 Mol Halogenierungsmittel und gegebenenfalls 0,001 bis 0,1 Mol, vorzugsweise 0,005 bis 0,05 Mol N,N-disubstituiertes Amid als Katalysator eingesetzt. Die Aufarbeitung und gegebenenfalls gewünschte Isolierung der Verbindungen der allgemeinen Formel (V) geschieht nach üblichen Methoden.

Als Beispiele für die erfindungsgemäß erhältlichen Verbindungen der Formel (V) seien die folgenden Verbindungen aufgeführt:

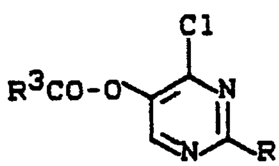

(V)

$R^3$= $CH_3$-, $C_2H_5$-, n-$C_3H_7$-, 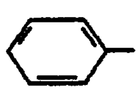 , $CH_3O$-, $C_2H_5O$-, n-$C_4H_9O$-

Tabelle 3

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 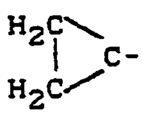 | 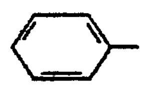 |
| 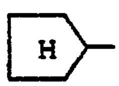 | 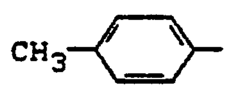 |
| 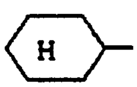 | |

Im Verfahrensschritt (c) werden die Verbindungen der allgemeinen Formel (V) gegebenenfalls ohne ihre Isolierung in die Verbindungen der allgemeinen Formel (VI) umgewandelt. Das Verfahren zur Herstellung der Verbindungen der Formel (VI) ist Teil der vorliegenden Erfindung.

Es wurde gefunden, daß man die 4-Chlor-5-hydroxy-pyrimidine der allgemeinen Formel (VI)

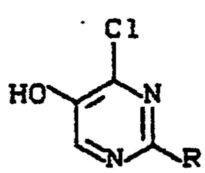 (VI)

in welcher

R die oben angegebenen Bedeutungen hat,

oder ihre Salze mit anorganischen Basen erhält, wenn man 4-Chlor-pyrimidin-Derivate der allgemeinen Formel (V)

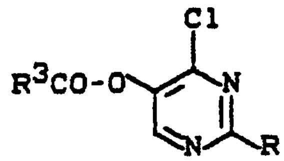

(V)

in welcher
R und $R^3$ die oben angegebenen Bedeutungen haben,
gegebenenfalls in Gegenwart von Verdünnungsmitteln, in Gegenwart von anorganischen Basen, bei Temperaturen zwischen 0° C und 160° C verseift und gegebenenfalls die Verbindungen der allgemeinen Formel (VI) durch Ansäuern freisetzt.

Überraschenderweise lassen sich die 4-Chlor-5-hydroxy-pyrmidine in guten Ausbeuten und in hoher Reinheit herstellen. Bei Kenntnis des Standes der Technik wäre eigentlich eine Halogensubstitution (vergl. z. B. "The Chemistry of Heterocyclic Compounds", Band The Pyrimidines/I, S. 148 - 149) derart zu erwarten gewesen, daß bei der basischen Hydrolyse die 4,5-Dihydroxy-pyrimidin-Derivate entstehen würden.

Verwendet man beispielsweise 4-Chlor-2-methyl-5-methylcarbonyloxy-pyrimidin als Ausgangsstoff und führt die Verseifung in Gegenwart von Natronlauge durch, so kann die Reaktion durch das folgende Formelschema skizziert werden:

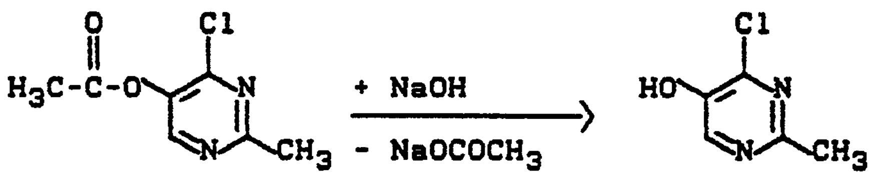


Das erfindungsgemäße Verfahren zur Herstellung der Verbindungen der Formel (VI) bzw. der Verfahrensschritt (c) wird bevorzugt in Gegenwart von Verdünnungsmitteln durchgeführt. Bevorzugt werden die Verdünnungsmittel eingesetzt, die bereits bei der Beschreibung des Verfahrensschrittes (b) angegeben worden sind.

Als anorganische Basen für das erfindungsgemäße Verfahren werden vorzugsweise verwendet: Alkali- und Erdalkalihydroxide wie z. B. Natrium-, Kalium- und Calcium-hydroxid, und Alkali-und Erdalkalicarbonate wie Natrium-, Kalium- und Calcium-carbonat.

Die Reaktionstemperatur kann innerhalb eines größeren Bereichs variiert werden. Im allgemeinen arbeitet man zwischen 0 °C und 160 °C, vorzugsweise bei 20 °C bis 140 °C. Das erfindungsgemäße Verfahren wird im allgemeinen bei Normaldruck durchgeführt.

Zur Durchführung des erfindungsgemäßen Verfahrens bzw. des Verfahrensschrittes (c) werden auf 1 Mol der Verbindung der Formel (V), 1 bis 5 Mol, vorzugsweise 2 bis 4,5 Mol Base eingesetzt. Die Aufarbeitung und gegebenenfalls gewünschte Isolierung der Verbindungen der Formel (VI) geschieht nach üblichen Methoden. Die Verbindungen der allgemeinen Formel (VI) fallen zunächst als Salze der verwendeten Basen an. Sie können direkt als Salze weiter umgesetzt werden. Es ist auch möglich durch Ansäuern, z.B. mit anorganischen Säuren, wie Salzsäure oder Schwefelsäure in üblicher Weise die Verbindungen der allgemeinen Formel (VI) freizusetzen.

Als Beispiele für die erfindungsgemäß erhältlichen Verbindungen der Formel (VI) seien die folgenden Verbindungen aufgefuhrt:

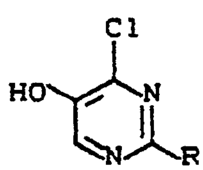

(VI)

Tabelle 4

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 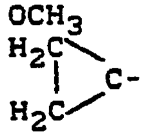 | 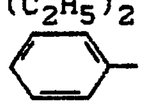 |
| 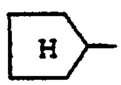 | 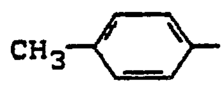 |
| 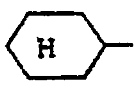 | |

sowie die Salze, insbesondere die Calcium-, Natrium- und Kaliumsalze dieser Verbindungen.

In der Verfahrensstufe (d) werden die Verbindungen der allgemeinen Formel (VII) oder ihre Salze (vorzugsweise Calcium-, Natrium- oder Kaliumsalze) aus den Verbindungen der Formel (VI) oder deren Salzen erhalten.

Verwendet man beispielsweise für die Verfahrensstufe (d) 4-Chlor-5-hydroxy-pyrimidin und Raney-Nickel als Katalysator, so kann die Reaktion durch das folgende Formelschema skizziert werden:

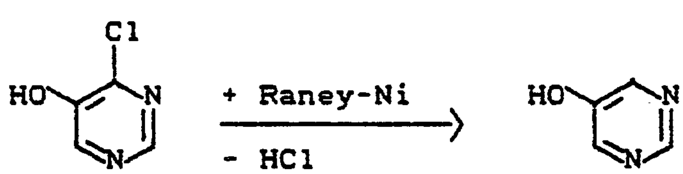


Für die Herstellung der Verbindungen der allgemeinen Formel (VII) oder ihren Salzen aus den Verbindungen der allgemeinen Formel (VI) oder deren Salzen werden die üblicherweise bei Hydrierungen verwendeten Lösungsmittel, wie Wasser, niedere aliphatische Alkohole oder Carbonsäuren, wie Methanol, Ethanol oder Essigsäure, vorzugsweise Wasser, verwendet.

Als Säureakzeptoren können alle üblicherweise verwendbaren anorganischen und organischen Basen verwendet werden. Hierzu gehören vorzugsweise Alkalicarbonate wie z. B. Natrium- und Kaliumcarbonat; Alkalihydroxide wie z. B. Natrium- und Kaliumhydroxid sowie niedere tertiäre Alkylamine, Cycloalkylamine und Aralkylamine, wie insbesondere Triethylamin.

Die Verfahrensstufe (d) wird in Gegenwart eines Hydrierungskatalysators durchgeführt. Es werden vorzugsweise neutrale Metallkatalysatoren wie Raney-Nickel, Raney-Cobalt oder Palladium gegebenenfalls auf üblichen Trägermaterialien wie z. B. Aktivkohle, eingesetzt.

Die Reaktionstemperaturen können in einem größeren Bereich variiert werden. Im allgemeinen arbeitet man zwischen 20 °C und 150 °C, vorzugsweise zwischen 20 °C und 100 °C, insbesondere zwischen 40 °C und 80 °C.

Die Verfahrensstufe (d) wird im allgemeinen bei erhöhtem Druck, vorzugsweise zwischen 5 und 100 bar, insbesondere zwischen 7 und 60 bar, durchgeführt.

Zur Durchführung der Verfahrensstufe (d) setzt man auf 1 Mol der Verbindung der Formel (VI) oder deren Salze zwischen 1 und 5 Mol, vorzugsweise zwischen 2 und 3 Mol Säureakzeptor und zwischen 1 und 100 g, vorzugsweise zwischen 5 und 50 g Katalysator ein.

Die Ausgangsstoffe der Formel (VI) oder deren Salze, der Säureakzeptor, der Katalysator und das Verdünnungsmittel werden vermischt und während des Aufheizens auf die erforderliche Temperatur wird Wasserstoff eingedrückt. Bei konstanter Temperatur wird so lange Wasserstoff eingedrückt, bis die Druckkonstanz das Reaktionsende anzeigt. Durch Ansäuern mit anorganischen Säuren (z.B. Salzsäure oder Schwefelsäure) können die Verbindungen der allgemeinen Formel (VII) in üblicher Weise freigesetzt werden.

Als Beispiele für die Verbindungen der allgemeinen Formel (VII) seien aufgeführt:

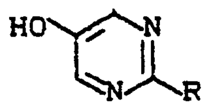

(VII)

Tabelle 5

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 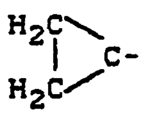 | 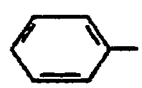 |
| 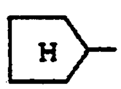 | 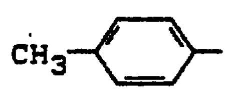 |
| 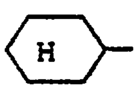 | |

sowie die Salze, insbesondere die Calcium-, Natrium- und Kaliumsalze dieser Verbindungen.

Bevorzugt werden die Verbindungen der Formel (VII) oder ihre Salze, ohne Isolierung der jeweiligen Zwischenprodukte der Formel (V) und (VI) und entsprechend den unter den Verfahrensschritten (b), (c) und (d) beschriebenen Reaktionsbedingungen aus den Verbindungen der Formel (IV) hergestellt (sog. "Eintopf-Reaktion").

Diese "Eintopf-Reaktion" ist Teil der vorliegenden Erfindung.

Überraschenderweise kann man nach dem erfindungsgemäßen "Eintopf"-Verfahren die 5-Hydroxy-pyrimidin-Derivate der allgemeinen Formel (VII) in guten Ausbeuten und in hoher Reinheit erhalten, obwohl zu erwarten war, daß eine Aneinanderreihung der obigen Reaktionsschritte ohne Isolierung und Reinigung der Zwischenprodukte nicht zu den gewünschten Produkten oder wegen Nebenreaktionen in einzelnen Stufen nur zu geringen Ausbeuten an verunreinigten Verbindungen führen würde.

In der Verfahrensstufe (e) werden die Verbindungen der allgemeinen Formel (I) aus den Verbindungen der allgemeinen Formeln (VII) und (VIII) erhalten. Die Verbindungen der allgemeinen Formel (VII) können hierbei auch in Form ihrer Salze eingesetzt werden.

Verwendet man in der Verfahrensstufe (e) beispielsweise O-Ethyl-O-isopropyl-thionophosphorsäurediesterchlorid und 5-Hydroxy-2-phenyl-pyrimidin als Ausgangsstoffe, so kann die entsprechende Reaktion durch das folgende Formelschema skizziert werden:

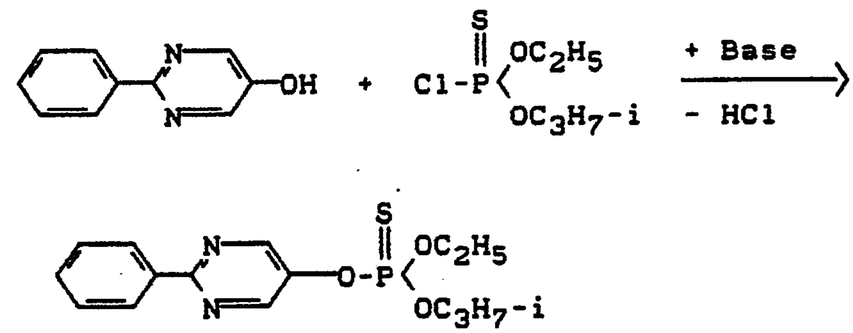


Die in der Verfahrensstufe (e) einzusetzenden Ausgangsstoffe der allgemeinen Formel (VIII) sind bekannt und nach literaturbekannten Verfahren und Methoden technisch gut herstellbar. Als Beispiele dafür seien im einzelnen genannt:
O,O-Dimethyl-, O,O-Diethyl-, O-O-Di-n-propyl, O,O-Di-isopropyl-, O,O-Di-n-butyl-, O,O-Di-iso-butyl-, O,O-Di-sec.-butyl-, O-Methyl-O-ethyl-, O-Methyl-O-n-propyl-, O-Methyl-O-iso-propyl-, O-Methyl-O-n-butyl-, O-Methyl-O-iso-butyl-, O-Methyl-O-seo.-butyl-, O-Ethyl-O-n-propyl-, O-Ethyl-O-isopropyl-, O-Ethyl-O-n-butyl-, O-Ethyl-O-sec.-butyl-, O-Ethyl-O-iso-butyl-, O-n-Propyl-O-butyl- bzw. O-iso-Propyl-O-butylphosphorsäurediesterchlorid und die entsprechenden Thionoanalogen, ferner O,S-Dimethyl-, O,S-Diethyl-, O,S-Di-n-propyl-, O,S-Di-iso-propyl-, O,S-Di-n-butyl-, O,S-Di-iso-butyl-, O-Ethyl-S-n-propyl-, O-Ethyl-S-iso-propyl-, O-Ethyl-S-n-butyl-, O-Ethyl-S-sec.-butyl-, O-n-Propyl-S-ethyl-, O-n-Propyl-S-iso-propyl-, O-n-Butyl-S-n-propyl und O-sec.-Butyl-S-ethylthiolphosphorsäurediesterchlorid und die entsprechenden Thioanalogen, ferner O-Methyl-, O-Ethyl-, O-n-Propyl-, O-iso-Propyl-, O-n-Butyl-, O-iso-Butyl- bzw. O-sec.-Butyl-methan- bzw. - ethan-, -n-propan-, -iso-propan-, -n-butan-, -sec.-butan- bzw. -phenyl-phosphorsäureesterchlorid und die entsprechenden Thioanalogen, und O-Methyl-N-methyl-, O-Methyl-N-ethyl-, O-Methyl-N-n-propyl-, O-Methyl-N-iso-propyl-, O-Ethyl-N-methyl-, O-Ethyl-N-ethyl-, O-Ethyl-N-n-propyl-, O-Ethyl-N-iso-propyl-, O-n-Propyl-N-methyl-, O-n-Propyl-N-ethyl-, O-n-Propyl-N-n-propyl-, O-n-Propyl-N-iso-propyl-, O-iso-Propyl-N-methyl-, O-iso-Propyl-N-ethyl-, O-iso-Propyl-N-n-propyl-, O-iso-Propyl-N-iso-propyl-, O-n-Butyl-N-methyl-, O-n-Butyl-N-ethyl-, O-n-Butyl-N-n-propyl-, O-n-Butyl-N-iso-propyl-, O-iso-Butyl-N-methyl-, O-iso-Butyl-N-ethyl-, O-iso-Butyl-N-n-propyl-, O-iso-Butyl-N-iso-propyl-, O-sec.-Butyl-N-methyl-, O-sec.-Butyl-N-ethyl-, O-sec.-Butyl-N-n-propyl- und O-sec.-Butyl-N-iso-propyl-phosphorsäuremonoesteramidchlorid und die entsprechenden Thionoanalogen. Besonders bevorzugt wird O-Ethyl-O-sec.-butyl-thionophosphorsäurediesterchlorid.

Die Verfahrensstufe (e) zur Herstellung der Verbindungen der allgemeinen Formel (I) wird bevorzugt unter Mitverwendung geeigneter Lösungs- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe wie Benzol, Toluol, Xylol, Benzin, Methylenchlorid, Chloroform, Tetrachlorkohlenstoff, Chlorbenzol oder Ether wie Diethyl-und Dibutylether, Dioxan, ferner Ketone, beispielsweise Aceton, Methylethyl-, Methylisopropyl- und Methylisobutylketon, außerdem Nitrile wie Aceto- und Propionitril.

Als Säureakzeptoren können alle üblichen Säurebindemittel Verwendung finden. Besonders bewährt haben sich Alkalicarbonate und -alkoholate wie Natrium- und Kaliumcarbonat, Kalium-tert.-butylat, ferner aliphatische, aromatische oder heterocyclische Amine, beispielsweise Triethylamin, Trimethylamin, Dimethylanilin, Dimethylbenzylamin und Pyridin. Falls die Verbindungen der allgemeinen Formel (VII) als Salze eingesetzt werden, ist die Zugabe des Säureakzeptors entbehrlich.

Die Verfahrensstufe (e) wird vorzugsweise in Gegenwart eines bicyclischen organischen Amins, wie Chinuclidin und 1,4-Diazabicyclo-(2,2,2)-octan (DABCO), vorzugsweise DABCO als Katalysator durchgeführt. Das bicyclische organische Amin kann zugleich auch als Säureakzeptor verwendet werden. In diesem Falle werden wenigstens molare Mengen des bicyclischen organischen Amins, insbesondere von DABCO eingesetzt.

In einer bevorzugten Ausführungsform werden als Säureakzeptoren Alkalicarbonate insbesondere $Na_2CO_3$ und/oder $K_2CO_3$ und als Katalysator katalytische Mengen DABCO eingesetzt.

Die Verfahrensstufe (e) wird im allgemeinen bei Temperaturen zwischen 0 °C und 120 °C durchgeführt. Bevorzugt ist der Temperaturbereich zwischen 20 °C und 100 °C. Die Umsetzungen werden im allgemeinen bei Normaldruck durchgeführt.

Zur Durchführung der Verfahrensstufe (e) werden auf 1 Mol der Verbindung der Formel (VII) oder des Salzes 1,0 bis 1,3 Mol, vorzugsweise zwischen 1,0 und 1,2 Mol (Thiono)-Phosphorsäurehalogenid der Formel (VIII) und im Falle der Verwendung eines bicyclischen Amins als Katalysator 0,01 bis 0,08 Mol, vorzugsweise zwischen 0,02 bis 0,06 Mol bi cyclisches organisches Amin (vorzugsweise DABCO) eingesetzt. Die Umsetzung wird im allgemeinen in einem Verdünnungsmittel sowie in Gegenwart eines Säureakzeptors durchgeführt. Der Säureakzeptor wird in Mengen zugefügt, welche geeignet sind, den

entsprechenden Halogenwasserstoff zu binden. Vorzugsweise werden je Mol Verbindung der Formel (VII) 0,8 bis 1,5, insbesondere 0,9 bis 1,3 und besonders bevorzugt 1,0 bis 1,2 Mol bzw. Äquivalent Säureakzeptor eingesetzt. Nach Ablauf der Reaktion wird filtriert und das Lösungsmittel im Vakuum abdestilliert.

Die Verbindungen der Formel (I) fallen in Form von Ölen an, die sich zum Teil nicht unzersetzt destillieren lassen, jedoch durch sogenanntes "Andestillieren", d. h. durch längeres Erhitzen unter vermindertem Druck auf mäßig erhöhte Temperaturen von den letzten flüchtigten Anteilen befreit und auf diese Weise gereinigt werden. Zu ihrer Charakterisierung dient der Brechungsindex.

Die erfindungsgemäß erhältlichen Verbindungen der allgemeinen Formel (I) zeichnen sich durch eine hervorragende insektizide, akarizide und nematizide Wirkung aus. Sie wirken gegen Pflanzen-, Hygiene- und Vorratsschädlinge und auf dem veterinärmedizinischen Sektor. Sie besitzen bei geringer Phytotoxizität sowohl eine gute Wirkung gegen saugende als auch fressende Insekten und Milben.

Aus diesem Grunde können die erfindungsgemäß erhältlichen Verbindungen der allgemeinen Formel (I) mit Erfolg im Pflanzenschutz sowie auf dem Hygiene-, Vorratsschutz- und Veterinärsektor als Schädlingsbekämpfungsmittel eingesetzt werden.

Die erfindungsgemäße erhältliche Verbindungen der Formel (I) können in bekannter Weise in den üblichen Formulierungen (mit 0,5 bis 95 % Wirkstoff), wie Stäubepulver, Granulaten, emulgierbaren Konzentraten, Spritzpulver oder Ultra-low-volume-Formulierungen in üblicher Weise, gegebenenfalls nach Verdünnung mit Wasser, auf die Pflanzen oder Böden ausgebracht werden. Je Hektar zu behandelnde Fläche werden zweckmäßigerweise etwa 0,1 bis 2,5 kg Wirkstoff eingesetzt.

Viele der erfindungsgemäß erhältlichen Verbindungen und ihre Verwendung sind bekannt und werden beschrieben z.B. in DE-OS 2 643 262, DE-OS 2 714 771, US-PS 4 127 652, DE-OS 3 326 510, EP-A 0 009 566, US-PS 4 325 948, US-PS 4 444 764, US-PS 4 429 125, US-PS 3 244 586 und DE-OS 3 317 824.

Wie bereits oben dargelegt, ist es nach dem erfindungsgemäßen Verfahren gemäß den Verfahrensstufen (a) bis (e) möglich, die wertvollen Verbindungen der allgemeinen Formel (I) in glatten Reaktionen auf einfache Weise herzustellen, wobei hervorragende Gesamtausbeuten erhalten werden. Das erfindungsgemäße Verfahren (a) bis (e) eröffnet in überrraschener Weise durch die spezielle Kombination der Verfahrensschritte und durch den teilweisen Einsatz hierbei entstehender neuer Verbindungen einen Weg, welcher eine bisher nicht erreichbare kostengünstige Herstellung der Verbindungen der allgemeinen Formel (I) erlaubt. Da die einzelnen Zwischenprodukte stabil sind und vor allem im Falle ihrer Isolierung über längere Zeit bevorratet werden können, erlaubt das erfindungsgemäße Verfahren darüber hinaus eine außerordentliche Flexibilität in der Produktion, so daß bei rasch einsetzendem Bedarf der Endprodukte eine bedarfsgerechte Fertigung möglich ist, was insbesondere durch die klimatisch bedingten starken saisonalen Schwankungen auf dem Pflanzenschutzgebiet von sehr großer Bedeutung sein kann.

Im folgenden sollen die erfindungsgemäßen Verfahren (bzw. Verfahrensstufen) und Verbindungen durch die nachstehenden Herstellungsbeispiele erläutert werden:

Verfahren zur Herstellung der Verbindungen der allgemeinen Formel (IV) (Verfahrensstufe (a))

Beispiel (IV-1)

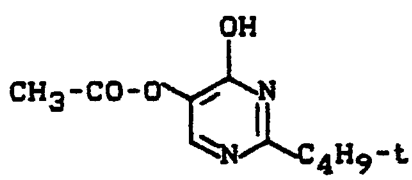

Ein Gemisch aus 16,8 g (0,1 Mol) 2-tert.-Butyl-4,5-dihydroxy-pyrimidin, 11,7 g (0,11 Mol) Natriumcarbonat und 100 ml Aceton wird mit 8,3 g (0,105 Mol) Acetylchlorid versetzt und 3 Stunden ohne Heizung nachgerührt. Dann saugt man vom anorganischen Salz ab, wäscht mit Aceton nach und dampft dann das Filtrat im Vakuum ein.

Man erhält so 18,4 g (88 % der Theorie) 2-tert.-Butyl-4-hydroxy-5-methylcarbonyloxy-pyrimidin in Form schwach gelber Kristalle vom Schmelzpunkt 164 °C.

Beispiel (IV-2)

Eine Mischung aus 84 g (0,5 Mol) 2-tert.-Butyl-,4,5-dihydroxy-pyrimidin, 350 ml Toluol und 56,5 g (0,55 Mol) Essigsäureanhydrid wird 6 Stunden unter Rückfluß gekocht. Dann destilliert man die flüchtigen Anteile im Vakuum vollständig ab.

Man erhält 102 g (98 % der Tehorie) 2-tert.-Butyl-4-hydroxy-5-methylcarbonyloxy-pyrimidin vom Schmelzpunkt 163 °C.

Analog Beispiel (IV-1) und (IV-2) können die folgenden Verbindungen der Formel (IV) hergestellt werden:

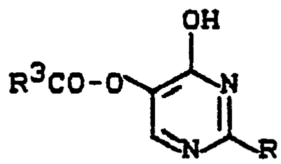

(IV)

Tabelle 6

| Beisp.-Nr. | R | $R^3$ | Acylierungsmittel der Formel (III) |
|---|---|---|---|
| (IV-3) | $C_4H_9$-tert. | $C_4H_9$-tert. | tert.-$C_4H_9COCl$ |
| (IV-4) | H | $CH_3$ | $CH_3COCl$ |
| (IV-5) | 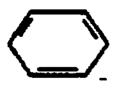 | $CH_3$ | $(CH_3CO)_2O$ |
| (IV-6) | 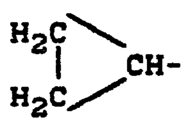 | $CH_3$ | $CH_3COCl$ |
| (IV-7) | $C_4H_9$-tert. | 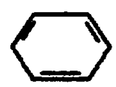 | 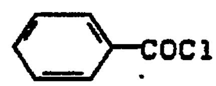 |
| (IV-8) | $OCH_3$ | $CH_3$ | $CH_3COCl$ |
| (IV-9) | $N(CH_3)_2$ | $CH_3$ | $CH_3COCl$ |

Verfahren zur Herstellung der Verbindungen der allgemeinen Formel (V) (Verfahrensstufe (b))

Beispiel (V-1)

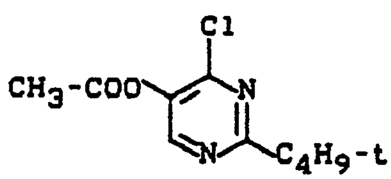

Eine Mischung aus 10,5 g (0,05 Mol) 2-tert.-Butyl-4-hydroxy-5-methylcarbonyloxy-pyrimidin und 20 ml Phosphorylchlorid wird 1/2 Stunde bei 60 - 70 °C gerührt und dann im Vakuum eingedampft. Den Rückstand versetzt man mit ca. 200 g Eis und extrahiert zweimal mit je 25 ml Methylenchlorid. Die vereinigten organischen Phasen trocknet man über Natriumsulfat, dann zieht man das Lösungsmittel im Vakuum ab und destilliert den Rückstand im Vakuum.

Man erhält so 6,8 g (60 % der Theorie) 2-tert.-Butyl-4-chlor-5-methylcarbonyloxy-pyrimidin als farbloses Öl mit dem Siedepunkt 64 °C/0,01 Torr.

Analog Beispiel (V-1) können die folgenden Verbindungen der Formel (V) hergestellt werden:

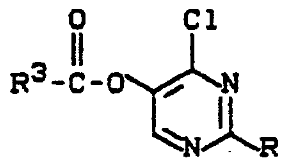

(V)

Tabelle 7

| Beisp.-Nr. | R | $R^3$ |
|---|---|---|
| (V-2) | $C_4H_9$-tert. | $C_4H_9$-tert. |
| (V-3) | H | $CH_3$ |
| (V-4) | 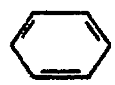 | $CH_3$ |
| (V-5) | 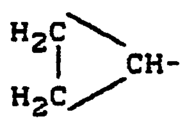 | $CH_3$ |
| (V-6) | $C_4H_9$-tert. | 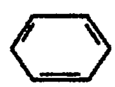 |
| (V-7) | $OCH_3$ | $CH_3$ |
| (V-8) | $N(CH_3)_2$ | $CH_3$ |
| (V-9) | $SCH_3$ | $CH_3$ |
| (V-10) | $C_4H_9$-tert. | $OCH_3$ |

Verfahren zur Herstellung der Verbindungen der allgemeinen Formel (VI) (Verfahrensstufe (c))

Beispiel (VI-1)

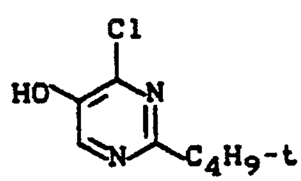

Zu einer Lösung von 11,5 g (0,05 Mol) 2-tert.-Butyl-4-chlor-5-methylcarbonyloxy-pyrimidin in 60 ml Toluol gibt man eine Mischung aus 15 g 45 proz. Natronlauge und 30 ml Wasser. Man rührt das Reaktionsgemisch 1 Stunde bei 90 °C, trennt die wäßrige Phase ab und befreit sie im Vakuum von Toluolresten. Dann wird unter Kühlen duch Zugabe von konz. Salzsäure auf pH 4 - 5 eingestellt. Das ausgefallene Produkt wird abgesaugt und mit Wasser nachgewaschen.

Man erhält so 8,6 g (92 % der Theorie) 2-tert.-Butyl-4-chlor-5-hydroxy-pyrimidin vom Schmelzpunkt 112 °C.

Beispiel (VI-2)

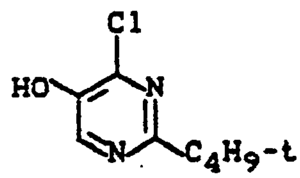

Durch 2 stündiges Erwärmen einer Mischung von 10,5 g (0,05 Mol) 2-tert.-Butyl-4-hydroxy-5-methylcarbonyloxy-pyrimidin, 50 ml Toluol, 0,1 ml Dimethylformamid und 30 ml einer 20 prozentigen Phosgenlösung in Toluol auf 60 - 70 °C wird eine Lösung von 2-tert.-Butyl-4-chlor-5-methylcarbonyloxy-pyrimidin hergestellt. Diese gibt man ohne weietre Reinigung zu einem Gemisch aus 17,5 g 45 proz. Natronlauge und 30 ml Wasser und kocht anschließend 1/2 Stunde unter Rückfluß nach. Die wäßrige Phase wird abgetrennt, im Vakuum von Toluolresten befreit und dann durch Zugabe von konz. Salzsäure auf pH 4 - 5 eingestellt. Man saugt das ausgefallene Produkt ab, wäscht mit Wasser nach und trocknet an der Luft.

Es werden 8,3 g (89 % der Theorie) 2-tert.-Butyl-4-chlor-5-hydroxy-pyrimidin in Form beiger Kristalle mit dem Schmelzpunkt 112 °C erhalten.

Analog Beispiel (VI-1) und (VI-2) können die folgenden Verbindungen der Formel (VI) erhalten werden:

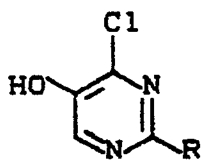

(VI)

**Tabelle 8**

| Beisp.-Nr. | R |
|---|---|
| (VI-3) | H |
| (VI-4) | 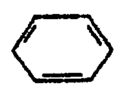 |
| (VI-5) | 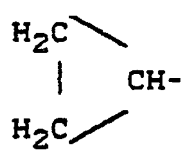 |
| (VI-6) | $OCH_3$ |
| (VI-7) | $N(CH_3)_2$ |
| (VI-8) | $SCH_3$ |
| (VI-9) | $i-C_3H_7$ |
| (VI-10) | $CH_3$ |

Verfahren zur Herstellung der Verbindungen der allgemeinen Formel (VII) (Verfahrensstufe (d))

Beispiel (VII-1)

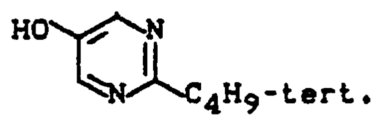

Eine Lösung von 186,5 g (1 Mol) 2-tert.-Butyl-4-chlor-5-hydroxy-pyrimidin und 84 g (2,1 Mol) Natriumhydroxid in 800 ml Wasser wird in Gegenwart von 15 g Raney-Nickel bei 50 °C und 10 bar Wasserstoffdruck hydriert. Nach Ende der Wasserstoffaufnahme wird der Katalysator abgesaugt. Das Filtrat wird mit konzentrierter Salzsäure versetzt bis pH 4 erreicht wird. Das ausgefallene Produkt wird abgesaugt und mit Wasser nachgewaschen.

Man erhält auf diese Weise 110 g (77% der Theorie) 2-tert.-Butyl-5-hydroxy-pyrimidin in Forme eines farblosen Pulvers mit dem Schmelzpunkt 132 °C.

Beispiel (VII-2) / "Eintopf-Verfahren"

Durch 6 stündiges Erwärmen einer Mischung aus 105 g (0,5 Mol) 2-tert.-Butyl-4-hydroxy-5-methylcarbonyloxy-pyrimidin, 450 ml Toluol, 1 ml Dimethylformamid und 70 g (0,55 Mol) Oxalylchlorid auf 60 °C wird eine Lösung von 2-tert.-Butyl-4-chlor-5-methylcarbonyloxy-pyrimidin hergestellt und zu einem Gemisch von 175 g 45 prozentiger Natronlauge und 300 ml Wasser gegeben. Man erwärmt 1 Stunde auf 65 °C, trennt die wäßrige Phase mit dem darin als Natriumsalz gelösten 2-tert.-Butyl-4-chlor-5-hydroxy-pyrimidin und hydriert sie bei 55 °C und 50 bar Wasserstoffdruck in Gegenwart von 75 g Raney-Nickel. Nach Abtrennen des Katalysators wird die Lösung mit konz. Salzsäure auf pH 4 - 5 eingestellt. Dann saugt man das ausgefallene Produkt ab, wäscht es mit Wasser nach und trocknet es bei 50 °C im Vakuum.

Man erhält auf diese Weise 52 g (68 % der Theorie) 2-tert.-Butyl-5-hydroxy-pyrimidin als beiges Pulver mit dem Schmelzpunkt 132 °C.

Analog Beispiel (VII-1) und (VII-2) können die folgenden Verbindungen der Formel (VII) erhalten werden:

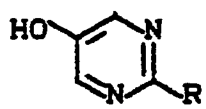

(VII)

**Tabelle 9**

| Beisp.-Nr. | R | Schmelzpunkt [°C] |
|---|---|---|
| (VII-3) | $C_3H_7$-n | 117 |
| (VII-4) | H | 216 |
| (VII-5) | $CH_3$ | 173 |
| (VII-6) | $N(CH_3)_2$ | 164 |
| (VII-7) | $C_2H_5$ | 149 |

Tabelle 9 - Fortsetzung

| Beisp.-Nr. | R | Schmelzpunkt [°C] |
|---|---|---|
| (VII-8) | 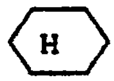 | 165 |
| (VII-9) | 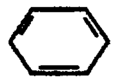 | 145 |
| (VII-10) | 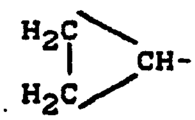 | |
| (VII-11) | $OCH_3$ | |
| (VII-12) | $SCH_3$ | |

Verfahren zur Herstellung der Verbindungen der allgemeinen Formel (I) (Verfahrensstufe (e))

Beispiel (I-1)

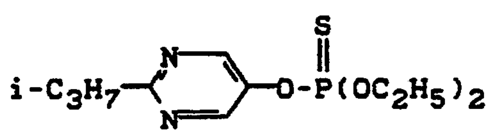

Ein Gemisch aus 300 ml Acetonitril, 13,8 g (0,1 Mol) 2-iso-Propyl-5-hydroxy-pyrimidin, 20,7 g (0,15 Mol) Kaliumcarbonat und 18,8 g (0,1 Mol) O,O-Diethyl-thionophosphorsäurediesterchlorid wird 2 Stunden bei 45 ° C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 17,4 g (62 % der Theorie) O,O-Diethyl-O-[2-iso-propyl-pyrimidin(5)yl]-thionophosphorsäureester in Form eines braunen Öls mit dem Brechungsindex $n_D^{21}$ : 1,4970

In analoger Weise können die folgende Verbindungen der Formel (I) hergestellt werden:

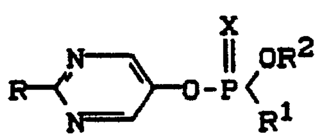 (I)

Tabelle 10

| Beisp.-Nr. | $R^2$ | $R^1$ | R | X | Ausbeute (% der Theorie) | Brechungsindex |
|---|---|---|---|---|---|---|
| (I-2) | $C_3H_7$-i | $CH_3$ | $C_3H_7$-i | S | 74 | $n_D^{21}$: 1,5102 |
| (I-3) | $CH_3$ | $OCH_3$ | $C_3H_7$-i | S | 66 | $n_D^{24}$: 1,5080 |
| (I-4) | $C_2H_5$ | $SC_3H_7$-n | $C_3H_7$-i | S | 69 | $n_D^{26}$: 1,5284 |
| (I-5) | $C_2H_5$ | 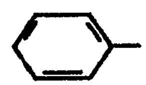 | $C_3H_7$-i | S | 74 | $n_D^{26}$: 1,5570 |
| (I-6) | $C_2H_5$ | $OC_2H_5$ | $C_3H_7$-i | O | 82 | $n_D^{32}$: 1,4630 |
| (I-7) | $C_2H_5$ | NH-$C_3H_7$-i | $C_3H_7$-i | S | 57 | $n_D^{32}$: 1,5057 |
| (I-8) | $C_3H_7$-n | $OC_2H_5$ | $C_3H_7$-i | S | 73 | $n_D^{32}$: 1,4929 |
| (I-9) | $C_2H_5$ | $OC_2H_5$ | $CH_3$ | S | 92 | $n_D^{32}$: 1,4992 |
| (I-10) | $C_2H_5$ | $C_2H_5$ | $CH_3$ | S | 80 | $n_D^{32}$: 1,5169 |

<u>Tabelle 10</u> - Fortsetzung

| Beisp.-Nr. | $R^2$ | $R^1$ | R | X | Ausbeute (% der Theorie) | Brechungsindex |
|---|---|---|---|---|---|---|
| (I-11) | $C_2H_5$ | $OC_2H_5$ | 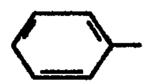 | S | 80 | $n_D^{32}$: 1,5643 |
| (I-12) | $C_2H_5$ | $C_2H_5$ | 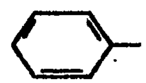 | S | 80 | $n_D^{32}$: 1,5827 |
| (I-13) | $C_2H_5$ | $OC_2H_5$ | H | S | 72 | $n_D^{32}$: 1,5028 |
| (I-14) | $C_2H_5$ | $OC_2H_5$ | $C_2H_5$ | S | 84 | $n_D^{20}$: 1,5014 |
| (I-15) | $C_2H_5$ | $OC_2H_5$ | $C_3H_7$-n | S | 60 | $n_D^{26}$: 1,4833 |
| (I-16) | $C_2H_5$ | $OC_2H_5$ | $C_4H_9$-n | S | 94 | $n_D^{21}$: 1,4958 |

Beispiel (I-17)

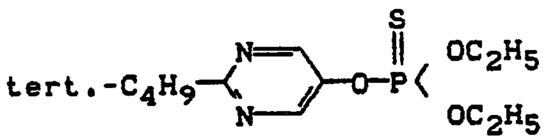

Zu einer Mischung aus 277 g (1,82 Mol) 2-tert.-Butyl-5-hydroxy-pyrimidin, 306,3 g (2,22 Mol) Kaliumcarbonat, 12,4 g (0,11 Mol) DABCO und 2 l Toluol gibt man auf einmal 344 g (1,82 Mol) O,O-Diethyl-thionophosphorsäurediesterchlorid. Die Temperatur steigt dabei bis auf ca. 35 °C an. Man rührt 4 Stunden ohne Heizung nach, saugt vom anorganischen Salz ab, wäscht mit Toluol nach und dampft dann das Filtrat im Vakuum ein. Der Rückstand wird bei 60 °C im Hochvakuum andestilliert.

Man erhält so 516 g (93 % der Theorie) O,O-Diethyl-O-(2-tert.-butyl-pyrimidin-5-yl)-thionophosphorsäureester vom Brechungsindex $n_D^{26}$: 1,4902.

Beispiel (I-18)

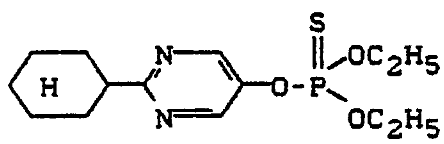

Ein Gemisch aus 300 ml Acetonitril, 17,8 g (0,1 Mol) 2-Cyclohexyl-5-hydroxy-pyrimidin, 20,7 g (0,15 Mol O,O-Diethylthionophosphorsäurediesterchlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 21,7 g (66 % der Theorie) O,O-Diethyl-O-(2-cyclohexyl-pyrimidin(5)yl)-thionophosphorsäureester in Form eines braunen Öles mit dem Brechungsindex $n_D^{23}$: 1,5158.

In analoger Weise können die folgenden Verbindungen der Formel (I) hergestellt werden:

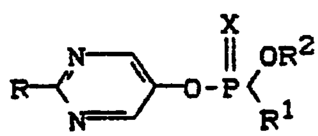

(I)

Tabelle 11

| Beisp.-Nr. | $R^2$ | $R^1$ | R | X | Ausbeute (% der Theorie) | Physikal. Daten (Brechungsindex; Schmelzpunkt °C) |
|---|---|---|---|---|---|---|
| (I-19) | $C_2H_5$ | $NH-C_3H_7-i$ | 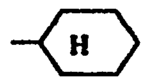 | S | 51 | $n_D^{23}$: 1,5246 |
| (I-20) | $CH_3$ | $OCH_3$ | 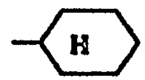 | S | 64 | $n_D^{23}$: 1,5287 |
| (I-21) | $C_2H_5$ | $OC_2H_5$ | 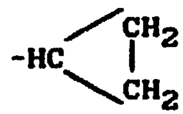 | S | 78 | $n_D^{24}$: 1,5142 |
| (I-22) | $C_2H_5$ | $NH-C_3H_7-i$ | 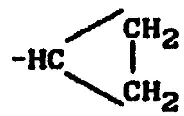 | S | 62 | 49 |
| (I-23) | $CH_3$ | $OCH_3$ | 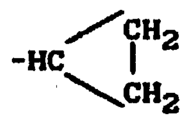 | S | 43 | $n_D^{24}$: 1,5390 |
| (I-24) | $C_3H_7-n$ | $OC_2H_5$ | 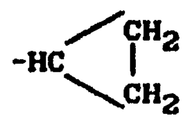 | S | 71 | $n_D^{25}$: 1,5128 |
| (I-25) | $C_2H_5$ | $NH-C_2H_5$ | 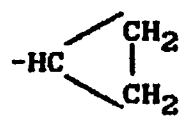 | S | 74 | $n_D^{26}$: 1,5310 |

<u>Tabelle 11</u> - Fortsetzung

| Beisp.-Nr. | $R^2$ | $R^1$ | R | X | Ausbeute (% der Theorie) | Physikal. Daten (Brechungsindex; Schmelzpunkt °C) |
|---|---|---|---|---|---|---|
| (I-26) | $C_2H_5$ | $OC_2H_5$ | 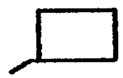 | S | | |
| (I-27) | $C_2H_5$ | $OC_2H_5$ | 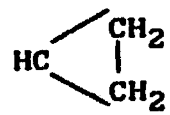 | S | | |
| (I-28) | $C_2H_5$ | $OC_2H_5$ | 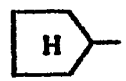 | S | 80 | $n_D^{23}$: 1,5164 |
| (I-29) | $C_2H_5$ | $OC_3H_7$-n | 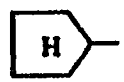 | S | | |
| (I-30) | $C_2H_5$ | $CH_3$ | 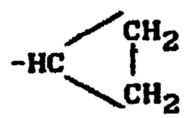 | S | 72 | $n_D^{25}$: 1,5428 |
| (I-31) | $C_2H_5$ | $OC_2H_5$ | 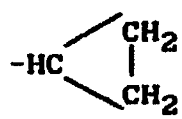 | O | | |
| (I-32) | $C_2H_5$ | NH-$C_3H_7$-i | 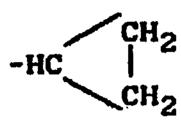 | O | | |

<u>Tabelle 11</u> - Fortsetzung

| Beisp.-Nr. | $R^2$ | $R^1$ | R | X | Ausbeute (% der Theorie) | Physikal. Daten (Brechungsindex; Schmelzpunkt °C) |
|---|---|---|---|---|---|---|
| (I-33) | $C_2H_5$ | 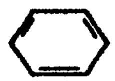 | 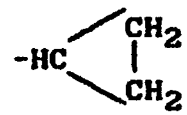 | S | 74 | $n_D^{25}$: 1,5815 |
| (I-34) | $C_2H_5$ | $SC_3H_7$-n | 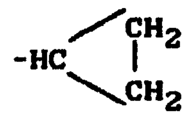 | S | | |
| (I-35) | $C_2H_5$ | 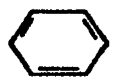 | H | S | | |
| (I-36) | $C_2H_5$ | $NH-C_2H_5$ | H | S | 66 | $n_D^{23}$: 1,5329 |
| (I-37) | $C_2H_5$ | $SC_3H_7$ | 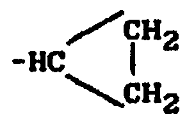 | O | | |
| (I-38) | $C_2H_5$ | $C_2H_5$ | 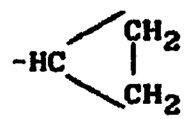 | S | | |
| (I-39) | $CH_3$ | $C_2H_5$ | 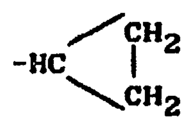 | S | | |

<u>Tabelle 11</u> - Fortsetzung

| Beisp.-Nr. | $R^2$ | $R^1$ | R | X | Ausbeute (% der Theorie) | Physikal. Daten (Brechungsindex; Schmelzpunkt °C) |
|---|---|---|---|---|---|---|
| (I-40) | $C_3H_7$-i | $CH_3$ | 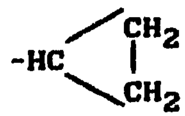 | S | 67 | $n_D^{26}$: 1,5233 |
| (I-41) | $CH_3$ | NH-$C_3H_7$-i | 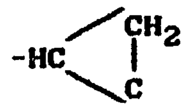 | S | | |
| (I-42) | $CH_3$ | NH-$CH_3$ | 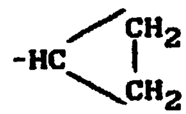 | S | 66 | $n_D^{26}$: 1,5460 |
| (I-43) | $C_2H_5$ | NH-$CH_3$ | 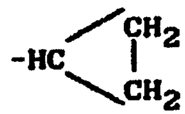 | S | | |
| (I-44) | $CH_3$ | NH-$C_2H_5$ | 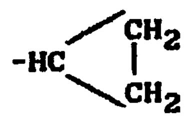 | S | | |
| (I-45) | $C_2H_5$ | NH-$C_3H_7$-i | 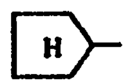 | S | 55 | $n_D^{23}$: 1,5247 |
| (I-46) | $C_2H_5$ | $OC_2H_5$ | 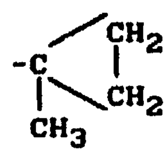 | S | | |

Beispiel (I-47)

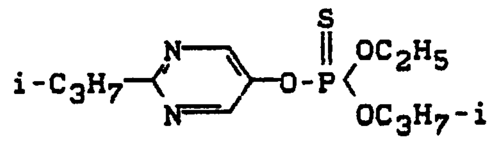

Ein Gemisch aus 300 ml Acetonitril, 13,8 g (0,1 Mol) 5-Hydroxy-2-iso-propyl-pyrimidin, 20,7 g (0,15 Mol) Kaliumcarbonat und 20,2 g (0,1 Mol) O-Ethyl-O-iso-propyl-thionophosphorsäurediester-chlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 28 g (92 % der Theorie) O-Ethyl-O-iso-propyl-O-(2-iso-propyl-pyrimidin-5-yl)-thionophosphorsäureester in Form eines gelben Öls mit dem Brechungsindex $n_D^{23}$: 1,4910.

In analoger Weise können die folgenden Verbindungen der Formel (I) hergestellt werden:

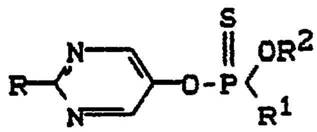

(I)

<u>Tabelle 12</u>

| Beisp.-Nr. | R | $R^2$ | $R^1$ | Brechungsindex |
|---|---|---|---|---|
| (I-48) | $C_3H_7$-i | $C_3H_7$-i | $OC_3H_7$-i | $n_D^{20}$: 1,4869 |
| (I-49) | $C_4H_9$-t | $C_2H_5$ | $OC_3H_7$-i | $n_D^{20}$: 1,4917 |
| (I-50) | $C_3H_7$-i | $C_2H_5$ | $OC_4H_9$-s | $n_D^{20}$: 1,4960 |
| (I-51) | $C_4H_9$-t | $C_2H_5$ | $OC_4H_9$-s | $n_D^{22}$: 1,4935 |
| (I-52) | $C_4H_9$-t | $C_3H_7$-i | $OC_3H_7$-i | $n_D^{22}$: 1,4857 |
| (I-53) | 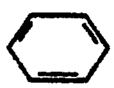 | $C_2H_5$ | $OC_3H_7$-i | $n_D^{22}$: 1,5516 |
| (I-54) | $C_4H_9$-t | $C_2H_5$ | $NHC_2H_5$ | $n_D^{21}$: 1,5100 |
| (I-55) | 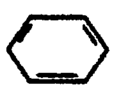 | $C_2H_5$ | $OC_4H_9$-s | |
| (I-56) | 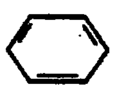 | $C_3H_7$-i | $OC_3H_7$-i | |
| (I-57) | $C_3H_7$-i | $C_3H_7$-n | $OC_3H_7$-n | $n_D^{23}$: 1,4915 |

**Patentansprüche**

1. Verfahren zur Herstellung von Verbindungen der allgemeinen Formel (I)

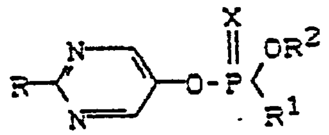

(I)

in welcher
R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$–$C_4$-Alkoxy oder $C_1$–$C_4$-Alkylsulfonyl substituiertes Alkyl mit 1 bis 12 Kohlenstoffatomen, für gegebenenfalls durch $C_1$–$C_4$-Alkyl substituiertes Cycloalkyl mit 3 bis 8 Kohlenstoffatomen, und für gegebenenfalls durch $C_1$–$C_4$-Alkyl, $C_1$–$C_4$-Alkoxy oder $C_1$–$C_4$-Alkylsulfonyl substituiertes Aryl mit 6 bis 10 Kohlenstoffatomen,
$R^1$ für gegebenenfalls durch $C_1$–$C_4$-Alkyl (gilt nicht, wenn $R^1$ für Alkyl steht), $C_1$–$C_4$-Alkoxy, $C_1$–$C_4$-Alkylthio, Halogen, Cyano und/oder Nitro substituierte Reste aus der Reihe $C_1$–$C_6$-Alkyl, $C_1$–$C_6$-Alkoxy, $C_1$–$C_6$-Alkylthio, Mono- oder Di-($C_1$–$C_5$)-alkylamino und Phenyl steht,
$R^2$ für gegebenenfalls durch $C_1$–$C_4$-Alkoxy, $C_1$–$C_4$-Alkylthio, Halogen, Cyano und/oder Nitro substituiertes $C_1$–$C_6$-Alkyl steht und
X für Sauerstoff oder Schwefel steht,
dadurch gekennzeichnet, daß man
a) Verbindungen der allgemeinen Formel (II)

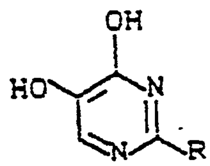

(II)

in welcher
R die oben angegebenen Bedeutungen hat, mit Acylierungsmitteln der Formel (III)

$R^3$–CO–Y (III)

in welcher
$R^3$ für $C_1$–$C_4$-Alkyl, $C_1$–$C_4$-Alkoxy oder Phenyl steht und
Y für Halogen oder eine Gruppierung –OCOR$^3$ steht,
gegebenenfalls in Gegenwart von Säureakzeptoren und gegebenenfalls in Gegenwart von Verdünnungsmitteln bei Temperaturen zwischen 0°C und 160°C zu den Verbindungen der allgemeinen Formel (IV)

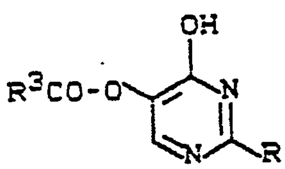

(IV)

in welcher
R und $R^3$ die oben angegebenen Bedeutungen haben,
umsetzt und anschließend
b) die Verbindungen der allgemeinen Formel (IV), gegebenenfalls nach ihrer Isolierung mit Halogenierungsmitteln gegebenenfalls in Gegenwart von N,N-disubstituierten Amiden als Katalysatoren und gegebenenfalls in Gegenwart von Verdünnungsmitteln bei Temperaturen zwischen 10°C und 120°C zu den Verbindungen der allgemeinen Formel (V)

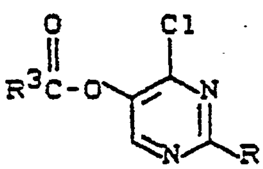

(V)

in welcher
R und $R^3$ die oben angegebenen Bedeutungen haben,
umsetzt und anschließend

c) die Verbindungen der allgemeinen Formel (V), gegebenenfalls nach ihrer Isolierung, in Gegenwart von anorganischen Basen bei Temperaturen zwischen 0°C und 160°C zu den Salzen der Verbindungen der allgemeinen Formel (VI)

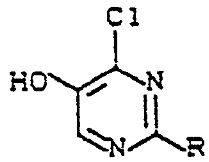

(VI)

in welcher
R die oben angegebene Bedeutung hat,
mit den verwendeten Basen umsetzt und gegebenenfalls nach Freisetzung der Verbindungen der allgemeinen Formel (VI) durch Ansäuren anschließend
d) die Verbindungen der allgemeinen Formel (VI) oder ihre Salze, gegebenenfalls nach ihrer Isolierung, mit Wasserstoff in Gegenwart von Hydrierungskatalysatoren, gegebenenfalls in Gegenwart von Säureakzeptoren und in Gegenwart von Verdünnungsmitteln, bei Temperaturen zwischen 20°C und 150°C zu den Verbindungen der allgemeinen Formel (VII)

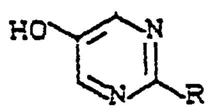

(VII)

in welcher
R die oben angegebene Bedeutung hat,
oder ihre Salze (mit den bei Stufe c) eingesetzten Basen) umsetzt und gegebenenfalls nach Freisetzung der Verbindungen der allgemeinen Formel (VII) durch Ansäuern anschließend
e) die Verbindungen der allgemeinen Formel (VII) oder ihre Salze gegebenenfalls nach ihrer Isolierung, mit Verbindungen der allgemeinen Formel (VIII)

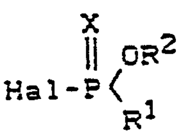

(VIII)

in welcher
Hal für Halogen steht und
X, $R^1$ und $R^2$ die oben angegebenen Bedeutungen haben,
gegebenenfalls in Gegenwart eines Säurebindemittels, gegebenenfalls in Gegenwart eines bicyclischen organischen Amins und gegebenenfalls in Gegenwart eines Lösungsmittels, umsetzt und die Verbindungen der allgemeinen Formel (I) isoliert.

2. Verfahren zur Herstellung von 4-Chlor-pyrimidin-Derivaten der allgemeinen Formel (V)

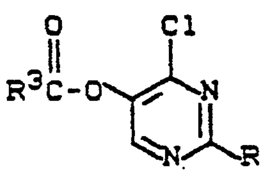

(V)

in welcher
R und $R^3$ die in Anspruch 1 angegebenen Bedeutungen haben,
dadurch gekennzeichnet, daß man 4-Hydroxy-pyrimidin-Derivate der allgemeinen Formel (IV)

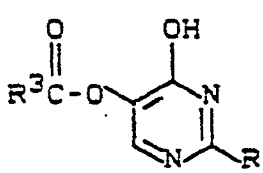

(IV)

in welcher
R und $R^3$ die in Anspruch 1 angegebenen Bedeutungen haben,
mit Halogenierungsmitteln gegebenenfalls in Gegenwart von N,N-disubstituierten Amiden als Katalysatoren und gegebenenfalls in Gegenwart von Verdünnungsmitteln bei Temperaturen zwischen 10°C und 120°C umsetzt.

3. Verfahren zur Herstellung von 4-Chlor-5-hydroxy-pyrimidinen der allgemeinen Formel (VI)

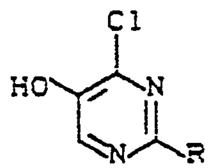

(VI)

in welcher

R die in Anspruch 1 angegebenen Bedeutungen hat,

oder ihrer Salze mit anorganischen Basen, dadurch gekennzeichnet, daß man 4-Chlor-pyrimidin-Derivate der allgemeinen Formel (V)

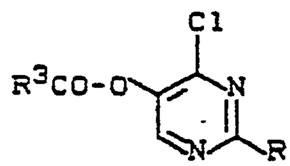

(V)

in welcher

R und $R^3$ die in Anspruch 1 angegebenen Bedeutungen haben,

gegebenenfalls in Gegenwart von Verdünnungsmitteln, in Gegenwart von anorganischen Basen, bei Temperaturen zwischen 0°C und 160°C verseift und gegebenenfalls die Verbindungen der allgemeinen Formel (VI) durch Ansäuern freisetzt.

4. Verfahren gemäß Anspruch 1, dadurch gekennzeichnet, daß die Verfahrensschritte b), c) und d) ohne die Isolierung der Zwischenprodukte ("Eintopfverfahren") durchgeführt werden.

5. Verfahren zur Herstellung von Verbindungen der allgemeinen Formel (VII)

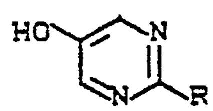

(VII)

in welcher

R die in Anspruch 1 angegebene Bedeutung hat,

dadurch gekennzeichnet, daß man die Verbindungen der allgemeinen Formel (IV)

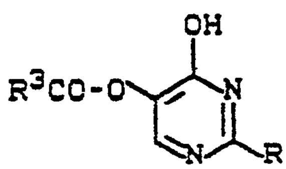

(IV)

in welcher

R und $R^3$ die in Anspruch 1 angegebenen Bedeutungen haben,

mit Halogenierungsmitteln gegebenenfalls in Gegenwart von N,N-disubstituierten Amiden als Katalysatoren und gegebenenfalls in Gegenwart von Verdünnungsmitteln bei Temperaturen zwischen 10°C und 120°C zu den Verbindungen der allgemeinen Formel (V)

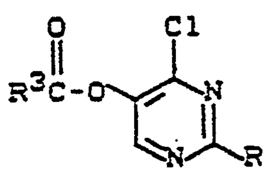

(V)

in welcher

R und $R^3$ die in Anspruch 1 angegebenen Bedeutungen haben,

umsetzt und anschließend

c) die Verbindungen der allgemeinen Formel (V), ohne ihre Isolierung, in Gegenwart von anorganischen Basen bei Temperaturen zwischen 0°C und 160°C zu den Salzen der Verbindungen der allgemeinen Formel (VI)

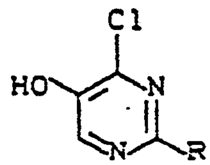

(VI)

in welcher
R die in Anspruch 1 angegebene Bedeutung hat,
mit den verwendeten Basen umsetzt und anschließend
d) die Salze der Verbindungen der allgemeinen Formel (VI), ohne ihre Isolierung, mit Wasserstoff in Gegenwart von Hydrierungskatalysatoren, gegebenenfalls in Gegenwart von Säureakzeptoren und in Gegenwart von Verdünnungsmitteln, bei Temperaturen zwischen 20°C und 150°C zu den Salzen der Verbindungen der allgemeinen Formel (VII)

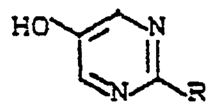

(VII)

in welcher
R die in Anspruch 1 angegebene Bedeutung hat,
umsetzt und anschließend die Verbindungen der allgemeinen Formel (VII) durch Ansäuern in üblicher Weise freisetzt und isoliert.

6. Verbindungen der allgemeinen Formel (IV)

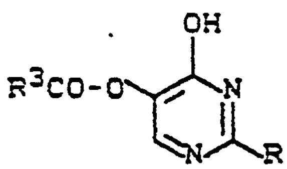

(IV)

in welcher
R und $R^3$ die in Anspruch 1 angegebenen Bedeutungen haben.

7. Verbindungen gemäß Anspruch 6, in welchen R für i.-$C_3H_7$ oder tert.-$C_4H_9$ (vorzugsweise tert.-$C_4H_9$) und $R^3$ für $C_1$–$C_4$-Alkyl (vorzugsweise Methyl) stehen.

8. Verbindungen der allgemeinen Formel (V)

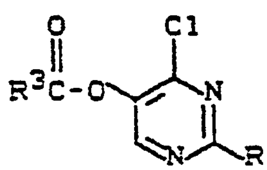

(V)

in welcher
R und $R^3$ die in Anspruch 1 angegebenen Bedeutungen haben.

9. Verbindungen gemäß Anspruch 8, in welchen R für i.-$C_3H_7$ oder tert.-$C_4H_9$ (vorzugsweise tert.-$C_4H_9$) und $R^3$ für $C_1$–$C_4$-Alkyl (vorzugsweise Methyl) stehen.

**Claims**

1. Process for the preparation of compounds of the general formula (I)

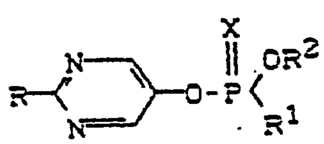

(I)

in which
R represents hydrogen, or represents alkoxy with 1 to 12 carbon atoms, or represents mono- or dialkylamino with in each case 1 to 6 carbon atoms in the alkyl part, or represents alkyl which has 1 to 12 carbon atoms and is optionally substituted by $C_1$–$C_4$-alkoxy or $C_1$–$C_4$-alkylsulphonyl, or represents cycloalkyl which has 3 to 8 carbon atoms and is optionally substituted by $C_1$–$C_4$-alkyl, or represents aryl which has 6 to 10 carbon atoms and is optionally substituted by $C_1$–$C_4$-alkyl, $C_1$–$C_4$-alkoxy or $C_1$–$C_4$-alkylsulphonyl,

$R^1$ represents radicals from the series comprising $C_1$–$C_6$-alkyl, $C_1$–$C_6$-alkoxy, $C_1$–$C_6$-alkylthio, mono- or di-($C_1$–$C_5$)-alkylamino and phenyl, which are optionally substituted by $C_1$–$C_4$-alkyl (does not apply if $R^1$ represents alkyl), $C_1$–$C_4$-alkoxy, $C_1$–$C_4$-alkylthio, halogen cyano and/or nitro,

$R^2$ represents $C_1$–$C_6$-alkyl optionally substituted by $C_1$–$C_4$-alkoxy, $C_1$–$C_4$-alkylthio, halogen, cyano and/or nitro and

X represents oxygen or sulphur,

characterized in that

a) compounds of the general formula (II)

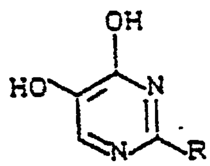

(II)

in which

R has the abovementioned meanings, are reacted with acylating agents of the formula (III)

$R^3$–CO–Y (III)

in which

$R^3$ represents $C_1$–$C_4$-alkyl, $C_1$–$C_4$-alkoxy or phenyl and

Y represents halogen or a grouping –$OCOR^3$, if appropriate in the presence of acid acceptors and if appropriate in the presence of diluents, at temperatures between 0°C and 160°C to give the compounds of the general formula (IV)

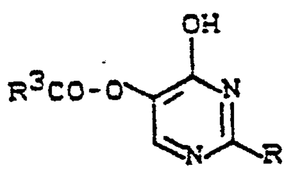

(IV)

in which

R and $R^3$ have the abovementioned meanings, and, subsequently,

b) the compounds of the general formula (IV), if appropriate after being isolated, are reacted with halogenating agents, if appropriate in the presence of N,N-disubstituted amides as catalysts and if appropriate in the presence of diluents, at temperatures between 10°C and 120°C to give the compounds of the general formula (V)

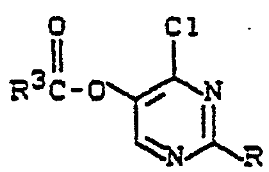

(V)

in which

R and $R^3$ have the abovementioned meanings, and, subsequently,

c) the compounds of the general formula (V), if appropriate after being isolated, are reacted in the presence of inorganic bases at temperatures between 0°C and 160°C to give the salts of the compounds of the general formula (VI)

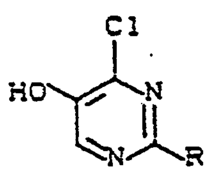

(VI)

in which

R has the abovementioned meaning,

with the bases used and, if appropriate, after liberation of the compounds of the general formula (VI) by acidification, subsequently

d) the compounds of the general formula (VI) or their salts, if appropriate after being isolated, are reacted with hydrogen in the presence of hydrogenation catalysts, if appropriate in the presence of acid acceptors and in the presence of diluents, at temperatures between 20°C and 150°C, to give the compounds of the general formula (VII)

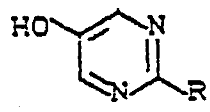

(VII)

in which
R has the abovementioned meaning,
or their salts (with the bases used in Stage c)), and, if appropriate after liberation of the compounds of the general formula (VII) by acidification, subsequently
e) the compounds of the general formula (VII) or their salts, if appropriate after being isolated, are reacted with compounds of the general formula (VIII)

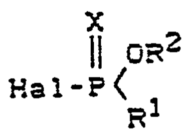

(VIII)

in which
Hal represents halogen and
X, $R^1$ and $R^2$ have the abovementioned meanings,
if appropriate in the presence of an acid-binding agent, if appropriate in the presence of a bicyclic organic amine and if appropriate in the presence of a solvent, and the compounds of the general formula (I) are isolated.

2. Process for the preparation of 4-chloro-pyrimidine derivatives of the general formula (V)

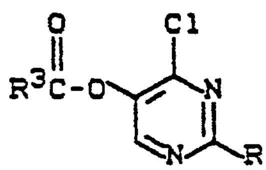

(V)

in which
R and $R^3$ have the meanings given in Claim 1,
characterized in that 4-hydroxy-pyrimidine derivatives of the general formula (IV)

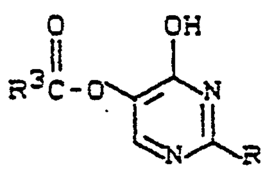

(IV)

in which
R and $R^3$ have the meanings given in Claim 1,
are reacted with halogenating agents, if appropriate in the presence of N,N-disubstituted amides as catalysts and if appropriate in the presence of diluents, at temperatures between 10°C and 120°C.

3. Process for the preparation of 4-chloro-5-hydroxy-pyrimidines of the general formula (VI)

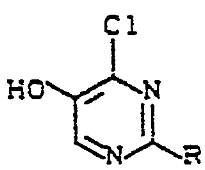

(VI)

in which
R has the meanings given in Claim 1,
or their salts with inorganic bases, characterized in that 4-chloro-pyrimidine derivatives of the general formula (V)

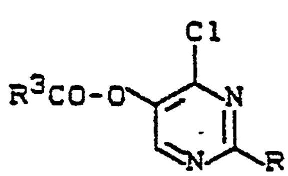

(V)

in which

R and $R^3$ have the meanings given in Claim 1,
are hydrolysed, if appropriate in the presence of diluents, in the presence of inorganic bases, at temperatures between 0°C and 160°C and, if appropriate, the compounds of the general formula (VI) are liberated by acidification.

4. Process according to Claim 1, characterized in that process steps b), c) and d) are carried out without isolation of the intermediate products ("one-pot process").

5. Process for the preparation of compounds of the general formula (VII)

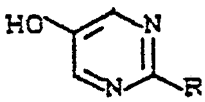

(VII)

in which
R has the meaning given in Claim 1,
characterized in that compounds of the general formula (IV)

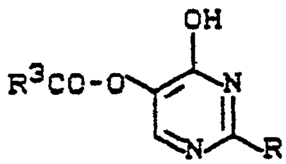

(IV)

in which
R and $R^3$ have the meanings given in Claim 1,
are reacted with halogenating agents, if appropriate in the presence of N,N-disubstituted amides as catalysts and if appropriate in the presence of diluents, at temperatures between 10°C and 120°C, to give the compounds of the general formula (V)

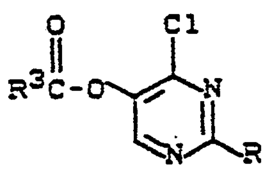

(V)

in which
R and $R^3$ have the meanings given in Claim 1,
and, subsequently,
c) the compounds of the general formula (V), without being isolated, are reacted in the presence of inorganic bases at temperatures between 0°C and 160°C to give the salts of the compounds of the general formula (VI)

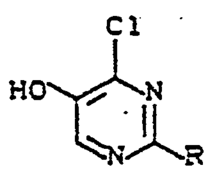

(VI)

in which
R has the meaning given in Claim 1,
with the bases used, and, subsequently,
d) the salts of the compounds of the general formula (VI), without being isolated, are reacted with hydrogen in the presence of hydrogenation catalysts, if appropriate in diluents, at temperatures between 20°C and 150°C, to give the salts of the compounds of the general formula (VII)

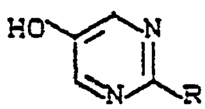

(VII)

in which
R has the meaning given in Claim 1,
and the compounds of the general formula (VII) are subsequently liberated by acidification in the customary manner and isolated.

6. Compounds of the general formula (IV)

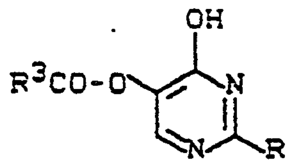

(IV)

in which

R and $R^3$ have the meanings given in Claim 1.

7. Compounds according to Claim 6, in which R represents i-$C_3H_7$ or tert.-$C_4H_9$ (preferably tert.-$C_4H_9$) and $R^3$ represents $C_1$–$C_4$-alkyl (preferably methyl).

8. Compounds of the general formula (V)

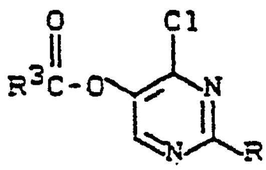

(V)

in which

R and $R^3$ have the meanings given in Claim 1.

9. Compounds according to Claim 8, in which R represents i-$C_3H_7$ or tert.-$C_4H_9$ (preferably tert.-$C_4H_9$) and $R^3$ represents $C_1$–$C_4$-alkyl (preferably methyl).

**Revendications**

1. Procédé de préparation de composés répondant à la formule générale (I)

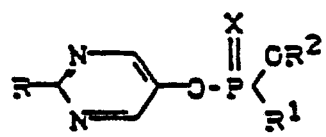

(I)

dans laquelle

R représente un atome d'hydrogène, un groupe alcoxy ayant 1 à 12 atomes de carbone, un groupe monoalkylamino ou dialkylamino ayant à chaque fois 1 à 6 atomes de carbone dans la partie alkylique, un groupe alkyle ayant 1 à 12 atomes de carbone (éventuellement substitué par un groupe alcoxy ayant 1 à 4 atomes de carbone ou par un groupe alkylsulfonyle ayant 1 à 4 atomes de carbone), un groupe cycloalkyle ayant 3 à 8 atomes de carbone (éventuellement substitué par un groupe alkyle ayant 1 à 4 atomes de carbone) et un groupe aryle ayant 6 à 10 atomes de carbone (éventuellement substitué par un groupe alkyle en $C_1$–$C_4$, alcoxy en $C_1$–$C_4$ ou alkylsulfonyle en $C_1$–$C_4$),

$R^1$ représente des restes choisis dans la série comportant un groupe alkyle en $C_1$–$C_6$, alcoxy en $C_1$–$C_6$, alkylthio en $C_1$–$C_6$, monoalkylamino ou dialkylamino (dont chaque groupe alkyle comporte 1 à 5 atomes de carbone) et phényle, ces restes étant éventuellement substitués par un groupe alkyle en $C_1$–$C_4$ (ce qui ne s'applique pas lorsque $R^1$ représente lui-même un groupe alkyle), alcoxy en $C_1$–$C_4$, alkylthio en $C_1$–$C_4$, halogéno, cyano et/ou nitro,

$R^2$ représente un groupe alkyle en $C_1$–$C_6$, éventuellement substitué par un groupe alcoxy en $C_1$–$C_4$, alkylthio en $C_1$–$C_4$, halogéno, cyano et/ou nitro, et

X représente un atome d'oxygène ou de soufre,

procédé caractérisé en ce que

a) on fait réagir des composés répondant à la formule générale (II):

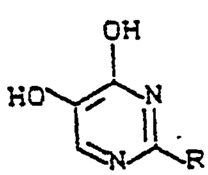

(II)

dans laquelle

R a les sens indiqués ci-dessus,

avec des agents d'acylation de formule (III)

$$R^3\text{–CO–Y} \quad \text{(III)}$$

dans laquelle

$R^3$ représente un groupe alkyle en $C_1$–$C_4$, alcoxy en $C_1$–$C_4$ ou phényle, et

Y représente un atome d'halogène ou un groupement –OCOR³, éventuellement en présence d'accepteurs d'acide et éventuellement en présence de diluants, en opérant à des températures comprises entre 0°C et 160°C, pour obtenir les composés de formule générale (IV):

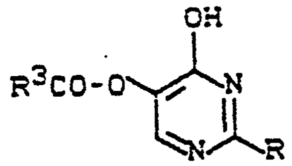

(IV)

dans laquelle
R et $R^3$ ont les sens indiqués ci-dessus, puis
b) on fait réagir les composés de formule générale (IV), éventuellement après leur isolement, avec des agents d'halogénation, en opérant éventuellement en présence d'amides N,N-disubstitués comme catalyseurs et éventuellement en présence de diluants, à des températures comprises entre 10°C et 120°C, pour obtenir les composés de formule générale (V):

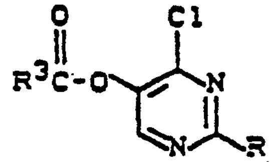

(V)

dans laquelle
R et $R^3$ ont les sens indiqués ci-dessus, puis
c) on fait réagir les composés de formule générale (V), éventuellement après leur isolement, en présence de bases minérales à des températures comprises entre 0°C et 160°C, pour obtenir les sels des composés de formule générale (VI):

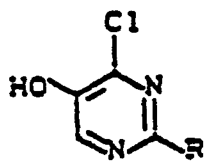

(VI)

dans laquelle
R a le sens indiqué ci-dessus,
avec les bases utilisées, et, éventuellement après mise en liberté des composés de formule générale (VI) par acidification,
d) on fait ensuite réagir les composés de formule générale (VI) ou leurs sels, éventuellement après leur isolement, avec de l'hydrogène en présence de catalyseurs d'hydrogénation, éventuellement en présence d'accepteurs d'acide et en présence de diluants, à des températures comprises entre 20°C et 150°C, pour obtenir les composés de formule générale (VII):

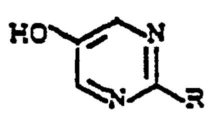

(VII)

dans laquelle
R a le sens indiqué ci-dessus,
ou leurs sels (avec les bases utilisées à l'étape c) et, éventuellement après mise en liberté, par acidification, des composés de formule générale (VII),
e) on fait ensuite réagir les composés de formule générale (VII) ou leurs sels, éventuellement après leur isolement, avec des composés de formule générale (VIII)

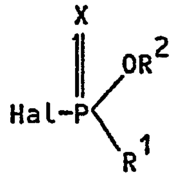

(VIII)

dans laquelle
Hal représente un atome d'halogène, et

X, $R^1$ et $R^2$ ont les sens indiqués ci-dessus, en opérant éventuellement en présence d'un agent de fixation des acides, éventuellement en présence d'une amine organique bicyclique et éventuellement en présence d'un solvant, et l'on isole les composés de formule générale (I).

2. Procédé de préparation des dérivés de la 4-chloro-pyrimidine de formule générale (V)

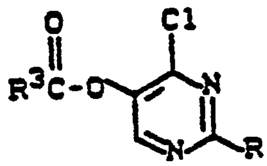

(V)

dans laquelle

R et $R^3$ ont les sens indiqués à la revendication 1,

procédé caractérisé en ce qu'on fait réagir, à des températures comprises entre 10°C et 120°C, des dérivés de la 4-hydroxy-pyrimidine de formule générale (IV)

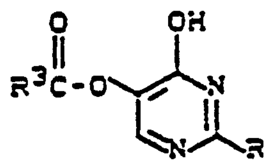

(IV)

dans laquelle

R et $R^3$ ont les sens indiqués à la revendication 1,

avec des agents d'halogénation, en opérant éventuellement en présence d'amides N,N-disubstitués comme catalyseurs et éventuellement en présence de diluants.

3. Procédé de préparation de 4-chloro-5-hydroxy-pyrimidines de formule générale (VI):

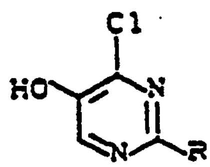

(VI)

dans laquelle

R a les sens indiqués à la revendication 1,

ou de leurs sels avec des bases minérales, procédé caractérisé en ce qu'on saponifie, à des températures comprises entre 0°C et 160°C, éventuellement en présence de diluants, en présence de bases organiques, des dérivés de la 4-chloro-pyrimidine de formule générale (V)

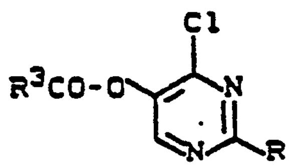

(V)

dans laquelle

R et $R^3$ ont les sens indiqués à la revendication 1,

et l'on met éventuellement en liberté, par début d'acidification, les composés de formule générale (VI).

4. Procédé selon la revendication 1, caractérisé en ce qu'on conduit les étapes opératoires b), c) et d), sans isoler les produits intermédiaires ("procédé conduit dans un seul récipient").

5. Procédé pour préparer des composés de formule générale (VII)

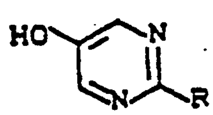

(VII)

dans laquelle

R a le sens indiqué à la revendication 1,

procédé caractérisé en ce qu'on fait réagir les composés de formule générale (IV)

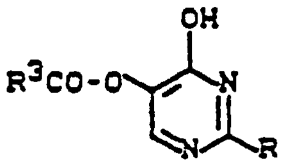

(IV)

dans laquelle

R et $R^3$ ont les sens indiqués à la revendication 1,

avec des agents d'halogénation, en opérant éventuellement en présence d'amides N,N-disubstitués comme catalyseurs et éventuellement en présence de diluants, à des températures comprises entre 10°C et 120°C, pour obtenir les composés de formule générale (V):

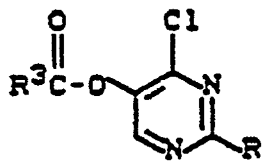

(V)

dans laquelle

R et $R^3$ ont les sens indiqués à la revendication 1, puis

c) on fait réagir les composés de formule générale (V), sans les isoler, en présence de bases minérales à des températures comprises entre 0°C et 160°C, pour obtenir les sels des composés de formule générale (VI)

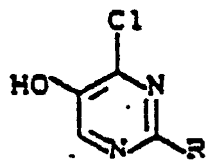

(VI)

dans laquelle

R a le sens indiqué à la revendication 1,

avec les bases utilisées, puis

d) on fait réagir les sels des composés de formule générale (VI), sans les isoler, avec de l'hydrogène en présence de catalyseurs d'hydrogénation, en opérant éventuellement en présence d'accepteurs d'acides et en présence de diluants, à des températures comprises entre 20°C et 150°C, pour obtenir les sels des composés de formule générale (VII)

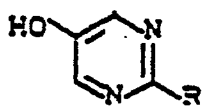

(VII)

dans laquelle

R a le sens indiqué à la revendication 1,

puis l'on met en liberté, par début d'acidification de la façon usuelle, les composés de formule générale (VII) que l'on isole.

6. Composés de formule générale (IV)

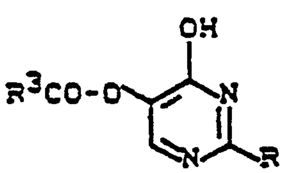

(IV)

dans laquelle

R et $R^3$ ont les sens indiqués à la revendication 1.

7. Composés selon la revendication 6, dans laquelle R représente un groupe iso-$C_3H_7$ ou tertio-$C_4H_9$ (de préférence un groupe tertio-$C_4H_9$) et $R^3$ représente un groupe alkyle en $C_1$–$C_4$ (de préférence un groupe méthyle).

8. Composés de formule générale (V):

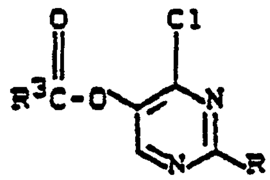

(V)

dans laquelle

R et $R^3$ ont les sens indiqués à la revendication 1.

9. Composés selon la revendication 8, dans lesquels R représente un groupe iso-$C_3H_7$ ou tertio-$C_4H_9$ (de préférence un groupe tertio-$C_4H_9$) et $R^3$ représente un groupe alkyle en $C_1$–$C_4$ (de préférence un groupe méthyle).